(19) Europäisches Patentamt
European Patent Office
Office européen des brevets

(11) EP 4 752 143 A2

(12) # EUROPEAN PATENT APPLICATION

(43) Date of publication:
**03.06.2026 Bulletin 2026/23**

(21) Application number: **26169908.6**

(22) Date of filing: **26.12.2022**

(51) International Patent Classification (IPC):
***C07D 403/14*** *(2006.01)*

(52) Cooperative Patent Classification (CPC):
**C07D 405/14; C07D 403/10; C07D 403/14; C09K 11/06; H10K 85/622; H10K 85/654; H10K 85/6572;** H10K 50/11; H10K 85/342; H10K 2101/10; H10K 2101/90; Y02E 10/549

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.12.2021 KR 20210193104**

(62) Document number(s) of the earlier application(s) in accordance with Art. 76 EPC:
**22916654.1 / 4 458 822**

(71) Applicant: **SAMSUNG SDI CO., LTD.**
**Yongin-si, Gyeonggi-do 17084 (KR)**

(72) Inventors:
- **LEE, Yoonman**
  **16678 Suwon-si, Gyeonggi-do (KR)**
- **GWAK, Seonyeong**
  **16678 Suwon-si, Gyeonggi-do (KR)**
- **LIM, Suyong**
  **16678 Suwon-si, Gyeonggi-do (KR)**
- **IM, Suheon**
  **16678 Suwon-si, Gyeonggi-do (KR)**
- **LEE, Mijin**
  **16778 Suwon-si, Gyeonggi-do (KR)**
- **LEE, Hanill**
  **16678 Suwon-si, Gyeonggi-do (KR)**
- **KIM, Wook**
  **16678 Suwon-si, Gyeonggi-do (KR)**

(74) Representative: **Michalski Hüttermann & Partner Patentanwälte mbB**
**Kaistraße 16A**
**40221 Düsseldorf (DE)**

Remarks:
This application was filed on 31.03.2026 as a divisional application to the application mentioned under INID code 62.

(54) **COMPOUND FOR ORGANIC OPTOELECTRONIC DEVICE, COMPOSITION FOR ORGANIC OPTOELECTRONIC DEVICE, ORGANIC OPTOELECTRONIC DEVICE, AND DISPLAY DEVICE**

(57) Disclosed are a compound for an organic optoelectronic device represented by Chemical Formula 1, a composition for an organic optoelectronic device including the same, an organic optoelectronic device, and a display device. The contents of Chemical Formula 1 are as defined in the detailed description.

【Figure 1】

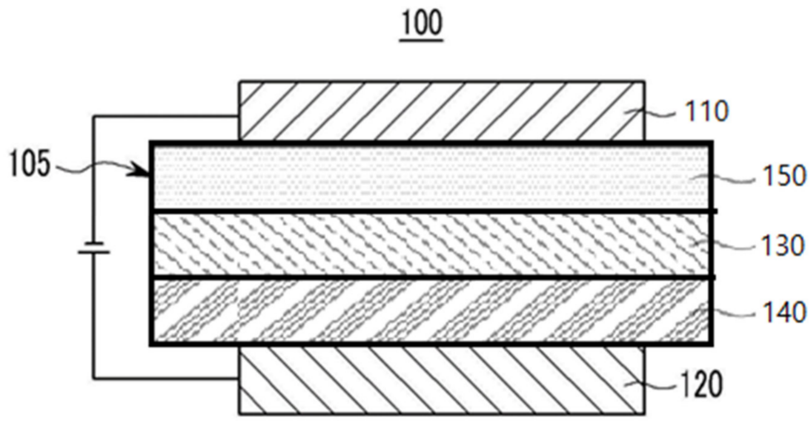




Processed by Luminess, 75001 PARIS (FR)

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photo conductor drum.

Among them, the organic light emitting diode (OLED) has recently drawn attention due to an increase in demand for flat panel displays. The organic light emitting diode converts electrical energy into light by applying current to an organic light emitting material and performance of an organic light emitting diode may be affected by organic materials disposed between electrodes.

### [Disclosure]

[Technical Problem]

An embodiment provides a compound for an organic optoelectronic device capable of implementing an organic optoelectronic device having low driving, high efficiency, and a long life-span.

Another embodiment provides a composition for an organic optoelectronic device capable of implementing an organic optoelectronic device having high efficiency and long life-span.

Another embodiment provides an organic optoelectronic device including the compound.

Another embodiment provides a display device including the organic optoelectronic device.

[Technical Solution]

According to an embodiment, a compound for an organic optoelectronic device represented by Chemical Formula 1 is provided.

[Chemical Formula 1]

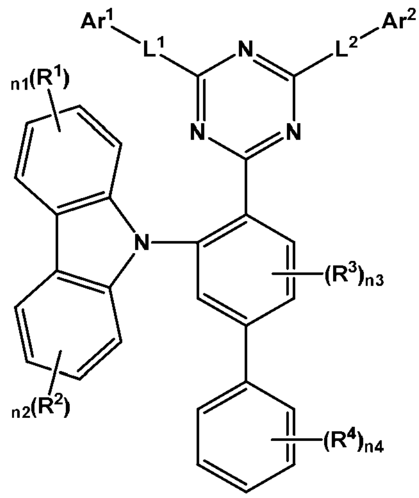

In Chemical Formula 1,

$Ar^1$ and $Ar^2$ are each independently a substituted or unsubstituted C6 to C30 aryl group, or a substituted or unsubstituted C2 to C30 heterocyclic group,
$L^1$ and $L^2$ are each independently a single bond, a substituted or unsubstituted C6 to C30 arylene group, or a substituted or unsubstituted C2 to C30 heteroarylene group,
$R^1$ and $R^2$ are each independently hydrogen, deuterium, or a substituted or unsubstituted C6 to C30 aryl group,
$R^3$ and $R^4$ are each independently hydrogen or deuterium,
n1, n2 and n4 are each independently one of integers of 1 to 4, and
n3 is one of integers of 1 to 3.

According to another embodiment, a composition for an organic optoelectronic device including a first compound and a second compound is provided.
The first compound is the same as described above and the second compound may be a compound for an organic optoelectronic device represented by Chemical Formula 2; or a compound for an organic optoelectronic device represented by a combination of Chemical Formula 3 and Chemical Formula 4.

[Chemical Formula 2]

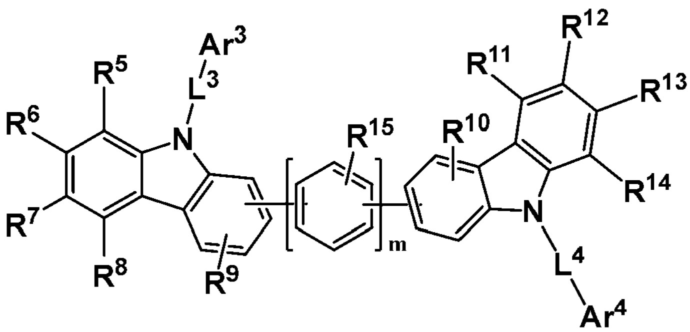

In Chemical Formula 2,

$Ar^3$ and $Ar^4$ are each independently a substituted or unsubstituted C6 to C20 aryl group or a substituted or unsubstituted C2 to C30 heterocyclic group,
$L^3$ and $L^4$ are each independently a single bond or a substituted or unsubstituted C6 to C20 arylene group,
$R^5$ to $R^{15}$ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group, and
m is one of integers of 0 to 2;

[Chemical Formula 3] [Chemical Formula 4]

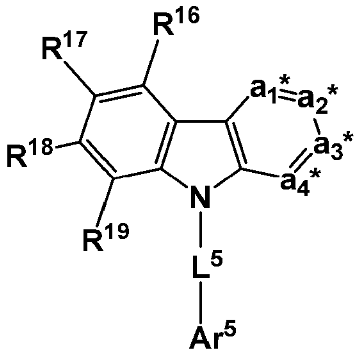

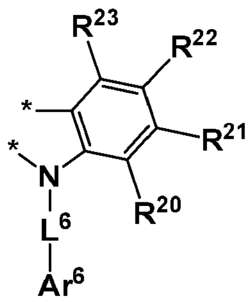

wherein, in Chemical Formulas 3 and 4,

$Ar^5$ and $Ar^6$ are each independently a substituted or unsubstituted C6 to C20 aryl group or a substituted or unsubstituted C2 to C30 heterocyclic group,

among $a_1$* to $a_4$* in Chemical Formula 3, two adjacent ones are each linking carbon (C) linked to * in Chemical Formula 4,

among $a_1$* to $a_4$* in Chemical Formula 3, the remaining two not linked to * in Chemical Formula 4 are each independently C-$L^a$-$R^a$,

$L^a$, $L^5$, and $L^6$ are each independently a single bond or a substituted or unsubstituted C6 to C20 arylene group, and

$R^a$ and $R^{16}$ to $R^{23}$ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

According to another embodiment, an organic optoelectronic device includes an anode and a cathode facing each other, and at least one organic layer between the anode and the cathode, wherein the organic layer includes a light emitting layer and the light emitting layer includes the aforementioned compound for an organic optoelectronic device or composition for an organic optoelectronic device.

According to another embodiment, a display device including the organic optoelectronic device is provided.

[Advantageous Effects]

An organic optoelectronic device having low driving, high efficiency, and a long life-span may be realized.

**[Description of the Drawings]**

FIG. 1 is a cross-sectional view illustrating an organic light emitting diode according to an embodiment.

<Description of Symbols>

100: organic light emitting diode
105: organic layer
110: cathode
120: anode
130: light emitting layer
140: hole transport region
150: electron transport region

**[Mode for Invention]**

Hereinafter, embodiments of the present invention are described in detail. However, these embodiments are

exemplary, the present invention is not limited thereto and the present invention is defined by the scope of claims.

As used herein, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In specific example of the present invention, the "substituted" refers to replacement of at least one hydrogen of a substituent or a compound by deuterium, a C1 to C20 alkyl group, a C6 to C30 aryl group, or a cyano group. In specific example of the present invention, the "substituted" refers to replacement of at least one hydrogen of a substituent or a compound by deuterium, a C1 to C5 alkyl group, a C6 to C18 aryl group, or a cyano group. In specific example of the present invention, the "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

"Unsubstituted" refers to non-replacement of a hydrogen atom by another substituent and remaining of the hydrogen atom.

In the present specification, "hydrogen substitution (-H)" may include "deuterium substitution (-D)" or "tritium substitution (-T)."

As used herein, when a definition is not otherwise provided, "hetero" refers to one including one to three heteroatoms selected from N, O, S, P, and Si, and remaining carbons in one functional group.

As used herein, "aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quaterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

As used herein, "a heterocyclic group" is a generic concept of a heteroaryl group, and may include at least one heteroatom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "a heteroaryl group" may refer to aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a

substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, or a combination thereof, but is not limited thereto.

As used herein, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

A compound for an organic optoelectronic device according to an embodiment is represented by Chemical Formula 1.

[Chemical Formula 1]

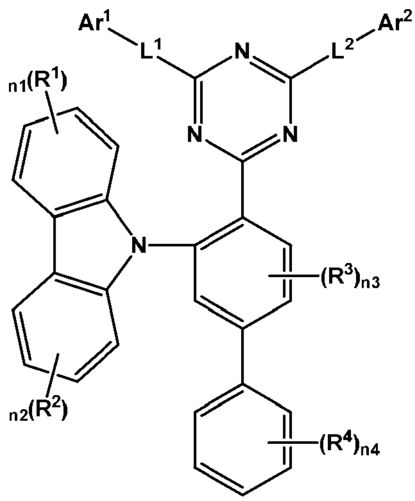

In Chemical Formula 1,

$Ar^1$ and $Ar^2$ are each independently a substituted or unsubstituted C6 to C30 aryl group, or a substituted or unsubstituted C2 to C30 heterocyclic group,
$L^1$ and $L^2$ are each independently a single bond, a substituted or unsubstituted C6 to C30 arylene group, or a substituted or unsubstituted C2 to C30 heteroarylene group,
$R^1$ and $R^2$ are each independently hydrogen, deuterium, or a substituted or unsubstituted C6 to C30 aryl group,
$R^3$ and $R^4$ are each independently hydrogen or deuterium,
n1, n2, and n4 are each independently one of integers of 1 to 4, and
n3 is one of integers of 1 to 3.

The compound represented by Chemical Formula 1 has a structure in which triazine is substituted with para-biphenyl substituted with ortho-carbazole, has particularly excellent energy transfer efficiency to a phosphorescent dopant, and thus it can be used as an advantageous material as a phosphorescent host.

The para-biphenyl structure included in Chemical Formula 1 can accelerate electron mobility compared to mono-phenyl and induce stability of electrons through resonance effect, thereby improving life-span, especially when applied as a phosphorescent host.

In addition, by substituting triazine and carbazole at the ortho position of the para-biphenyl, a dihedral angle increases due to steric hindrance between triazine and carbazole, and the triazine moiety and the carbazole moiety are twisted together.

This means that the HOMO energy level and the LUMO energy level are mostly separated without overlap, and fast energy transfer is possible using a small △Est, and thus it shows high efficiency characteristics, especially when applied as a phosphorescent host.

In addition, the side reaction path in the excited state is reduced, which increases the life-span, especially when applied as a phosphorescent host.

On the other hand, ortho substitution of triazine and carbazole can minimize degradation and decomposition by

reducing the deposition temperature by about 10% compared to para substitution and meta substitution.

When n1 is 2 or more, each $R^1$ may be the same or different from each other.

When n2 is 2 or more, each $R^2$ may be the same or different from each other.

When n3 is 2 or more, each $R^3$ may be the same or different from each other.

When n4 is 2 or more, each $R^4$ may be the same or different from each other.

Chemical Formula 1 may be represented by any one of Chemical Formula 1-1 to Chemical Formula 1-4.

[Chemical Formula 1-1] [Chemical Formula 1-2]

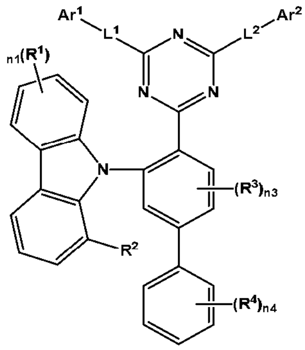

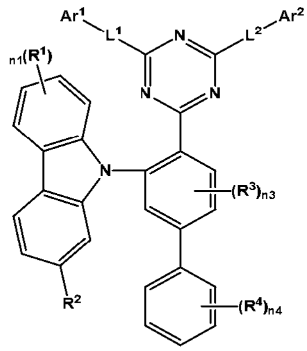

[Chemical Formula 1-3] [Chemical Formula 1-4]

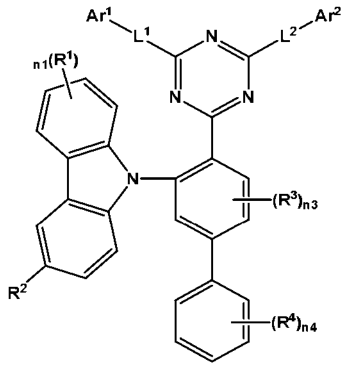

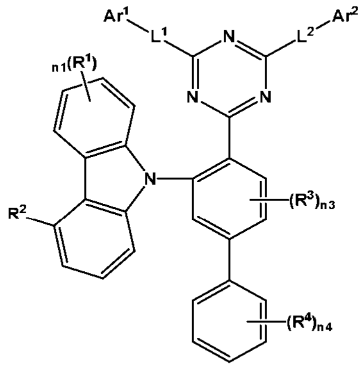

In Chemical Formula 1-1 to Chemical Formula 1-4,
the definitions of $Ar^1$ to $Ar^3$, $R^1$ to $R^4$, n1 to n4, $L^1$, and $L^2$ are the same as described above.

For example, Chemical Formula 1 may be represented by Chemical Formula 1-2.

For example, $R^1$ and $R^2$ may each independently be hydrogen, deuterium, a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, or a substituted or unsubstituted triphenylene group.

As a specific example, $R^1$ and $R^2$ may each independently be hydrogen, deuterium, a substituted or unsubstituted phenyl group, a substituted or unsubstituted biphenyl group, or a substituted or unsubstituted naphthyl group.

For example, $R^1$ and $R^2$ may each independently be hydrogen, deuterium or unsubstituted phenyl group.

For example, $Ar^1$ and $Ar^2$ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylene group, a substituted or unsubstituted carbazolyl group, a substituted or unsubstituted dibenzofuranyl group, or a substituted or unsubstituted dibenzothiophenyl group.

As a specific example, for example, *-$L^1$-$Ar^1$ and *-$L^2$-$Ar^2$ may each independently be selected from the substituents listed in Group I .

[Group Ⅰ]

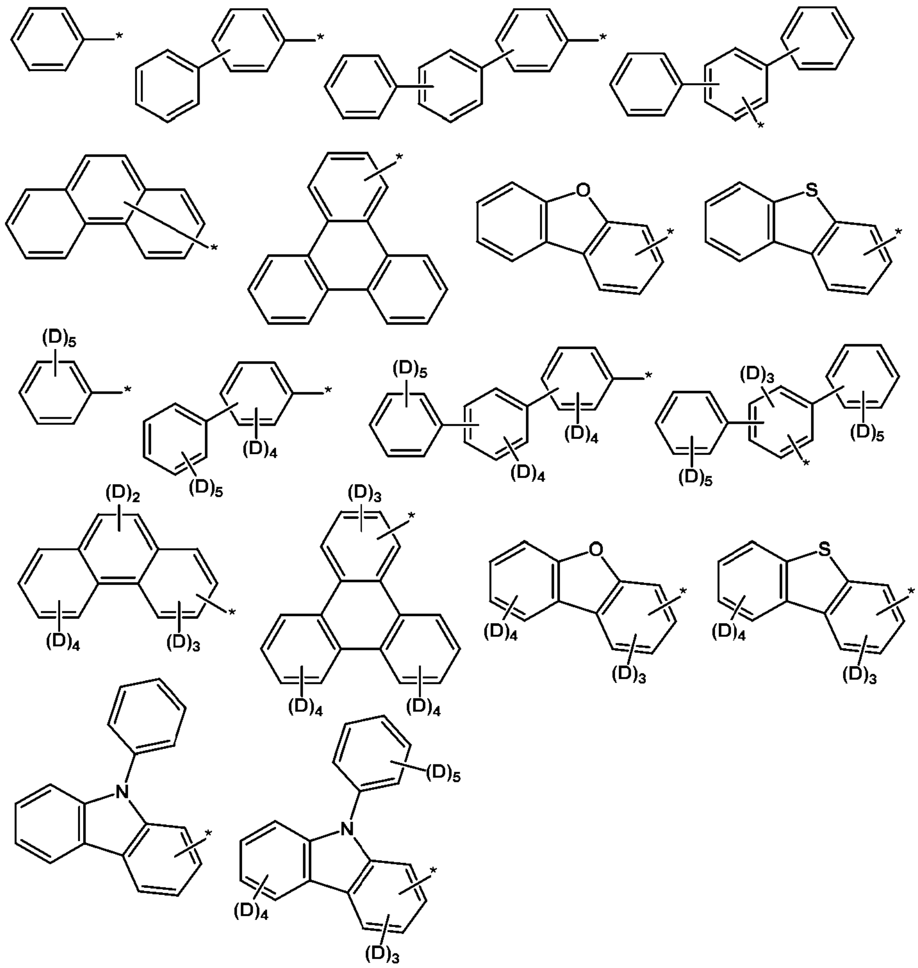

In Group I, * is a linking point.

For example, $Ar^1$ and $Ar^2$ may each independently be a substituted or unsubstituted phenyl group, a substituted or unsubstituted biphenyl group, a substituted or unsubstituted naphthyl group, a substituted or unsubstituted anthracenyl group, a substituted or unsubstituted phenanthrenyl group, or a substituted or unsubstituted triphenylene group.

In the most specific embodiment, the compound for an organic optoelectronic device represented by Chemical Formula 1 may be one selected from the compounds listed in Group 1, but is not limited thereto.

[Group 1]

[A-1] [A-2] [A-3] [A-4] [A-5]

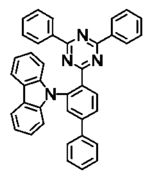 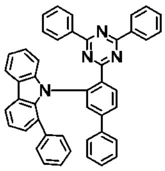 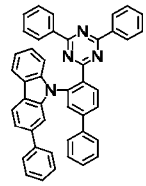 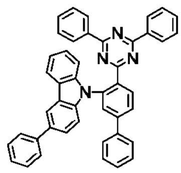 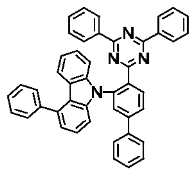

[A-6] [A-7] [A-8] [A-9] [A-10]

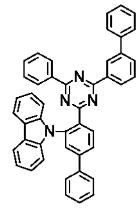 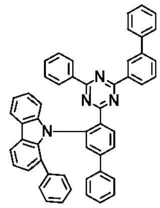 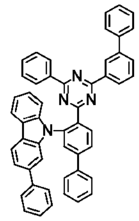 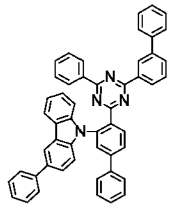 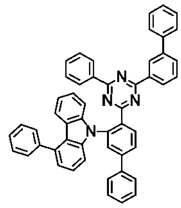

[A-11] [A-12] [A-13] [A-14] [A-15]

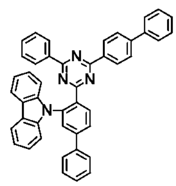 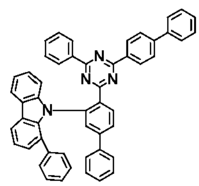 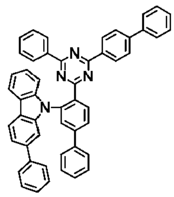 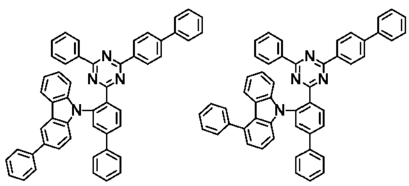

[A-16] [A-17] [A-18] [A-19] [A-20]

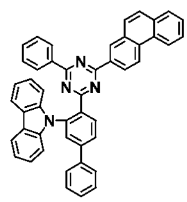 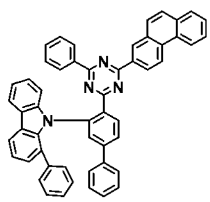 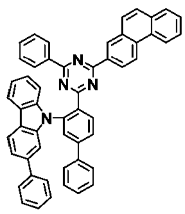 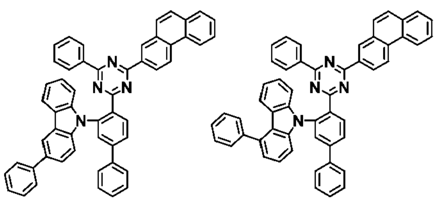

[A-21] [A-22] [A-23] [A-24] [A-25]

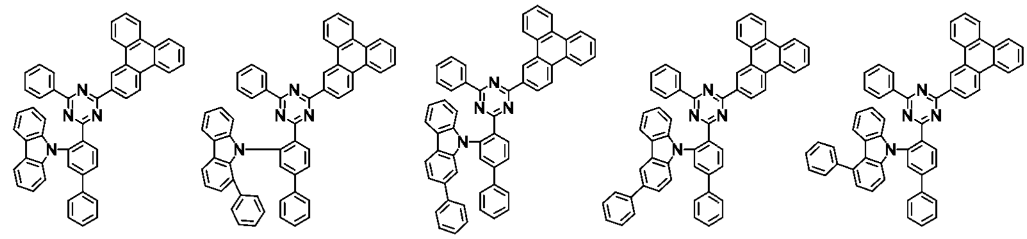

[A-26] [A-27] [A-28] [A-29] [A-30]

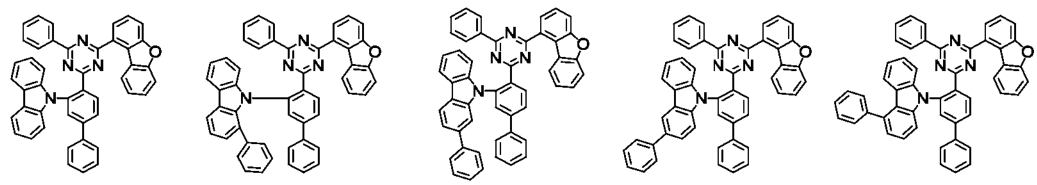

[A-31] [A-32] [A-33] [A-34] [A-35]

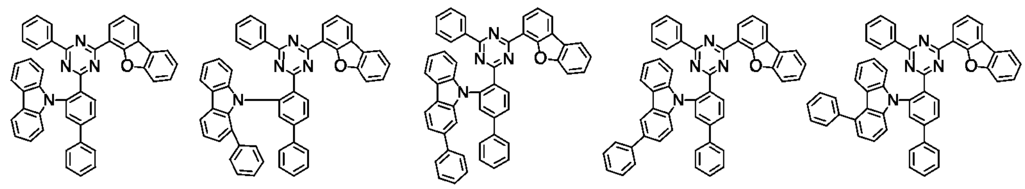

[A-36] [A-37] [A-38] [A-39] [A-40]

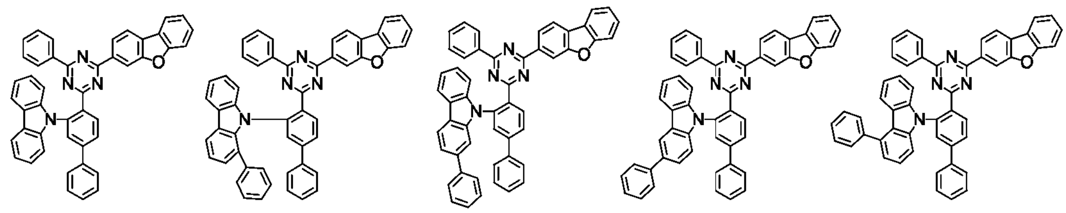

[A-41] [A-42] [A-43] [A-44] [A-45]

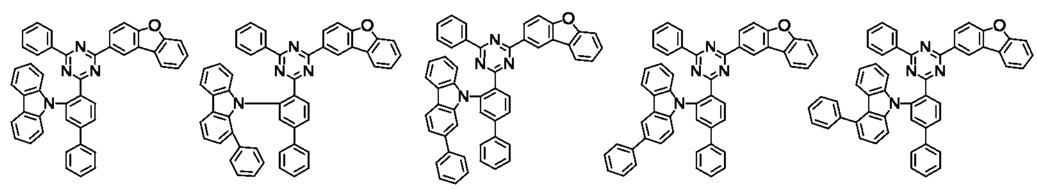

[A-46] [A-47] [A-48] [A-49] [A-50]

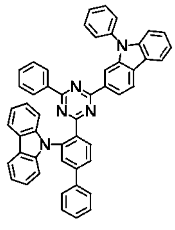 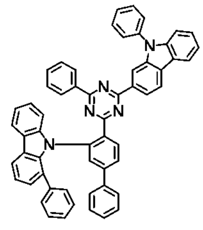 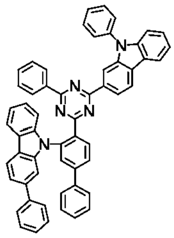 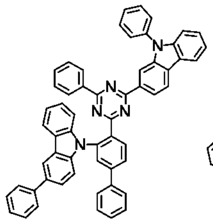 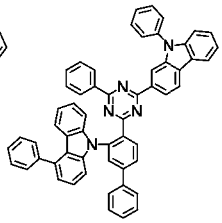

[A-51] [A-52] [A-53] [A-54] [A-55]

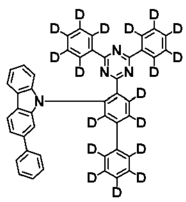 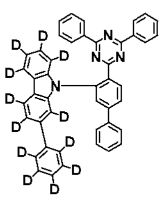 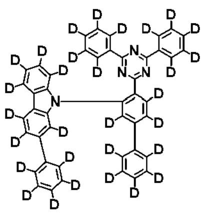 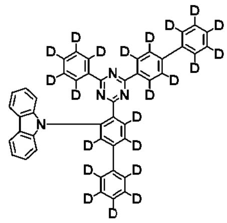 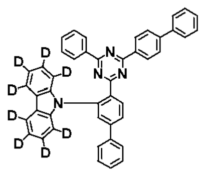

[A-56] [A-57] [A-58] [A-59] [A-60]

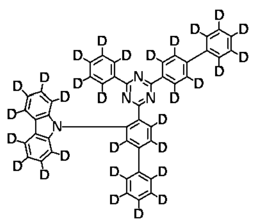 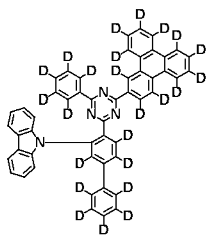 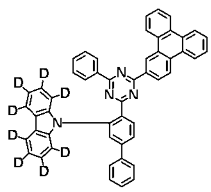 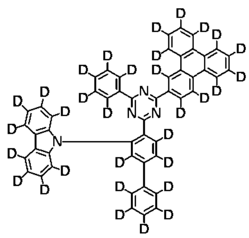

A composition for an organic optoelectronic device according to another embodiment includes a first compound and a second compound, wherein the first compound is the aforementioned compound for an organic optoelectronic device, and the second compound may be a compound for an organic optoelectronic device represented by Chemical Formula 2: or a compound for an organic optoelectronic device represented by a combination of Chemical Formula 3 and Chemical Formula 4.

[Chemical Formula 2]

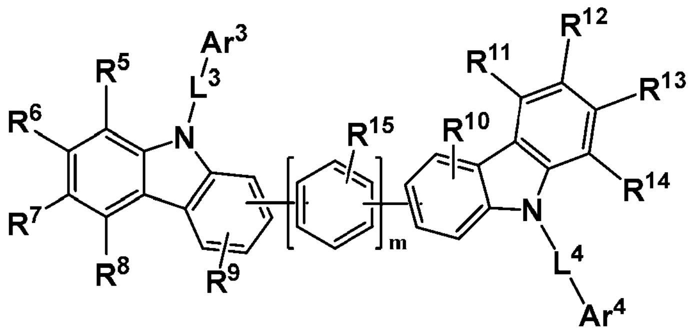

In Chemical Formula 2,

$Ar^3$ and $Ar^4$ are each independently a substituted or unsubstituted C6 to C20 aryl group or a substituted or unsubstituted C2 to C30 heterocyclic group,

$L^3$ and $L^4$ are each independently a single bond or a substituted or unsubstituted C6 to C20 arylene group,

$R^5$ to $R^{15}$ are each independently hydrogen, deuterium, a cyano group, a halogen, a substituted or unsubstituted

amine group, a substituted or unsubstituted C1 to C30 alkyl group, a substituted or unsubstituted C6 to C30 aryl group, or a substituted or unsubstituted C2 to C30 heterocyclic group, and
m is one of integers of 0 to 2;

[Chemical Formula 3] [Chemical Formula 4]

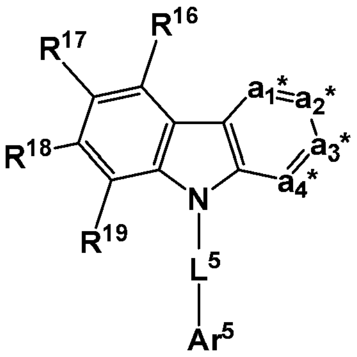

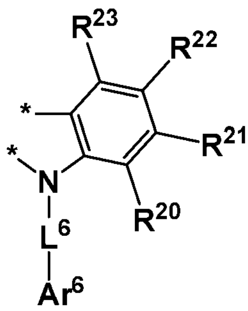

wherein, in Chemical Formulas 3 and 4,
$Ar^5$ and $Ar^6$ are each independently a substituted or unsubstituted C6 to C20 aryl group or a substituted or unsubstituted C2 to C30 heterocyclic group,
among $a_1$* to $a_4$* in Chemical Formula 3, two adjacent ones are each linking carbon (C) linked to * in Chemical Formula 4,
among $a_1$* to $a_4$* in Chemical Formula 3, the remaining two not linked to * in Chemical Formula 4 are each independently C-$L^a$-$R^a$,
$L^a$, $L^5$, and $L^6$ are each independently a single bond or a substituted or unsubstituted C6 to C20 arylene group, and
$R^a$ and $R^{16}$ to $R^{23}$ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

The second compound can be used in the light emitting layer together with the first compound to improve luminous efficiency and life-span characteristics by increasing charge mobility and stability.

For example, in Chemical Formula 2, $Ar^3$ and $Ar^4$ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,

in Chemical Formula 2, $L^3$ and $L^4$ may each independently be a single bond, a substituted or unsubstituted phenylene group, or a substituted or unsubstituted biphenylene group, and
in Chemical Formula 2, $R^5$ to $R^{15}$ may each independently be hydrogen, deuterium, or a substituted or unsubstituted C6 to C12 aryl group,
m may be 0 or 1.

As an example, in Chemical Formula 2, "substituted" refers to replacement of at least one hydrogen by deuterium, a C1 to C4 alkyl group, a C6 to C18 aryl group, or a C2 to C30 heteroaryl group.

In a specific embodiment of the present invention, Chemical Formula 2 may be expressed as one of Chemical Formula 2-1 to Chemical Formula 2-15.

[Chemical Formula 2-1] [Chemical Formula 2-2] [Chemical Formula 2-3]

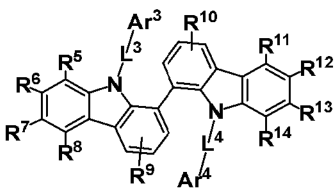
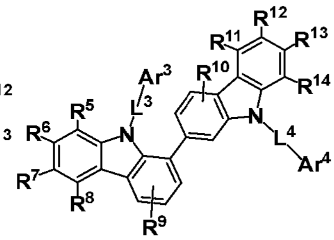
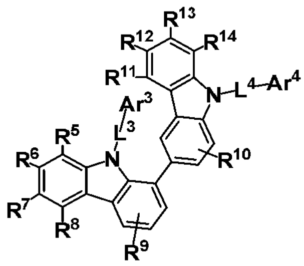

[Chemical Formula 2-4] [Chemical Formula 2-5] [Chemical Formula 2-6]

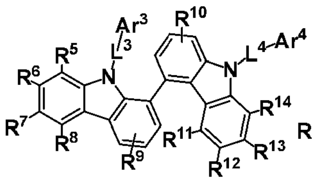
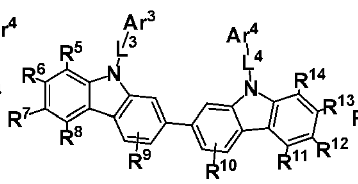
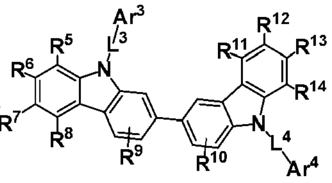

[Chemical Formula 2-7] [Chemical Formula 2-8] [Chemical Formula 2-9]

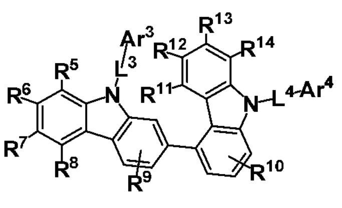
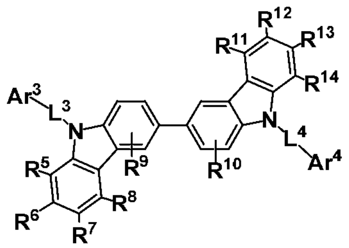
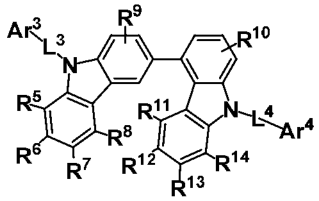

[Chemical Formula 2-10] [Chemical Formula 2-11] [Chemical Formula 2-12]

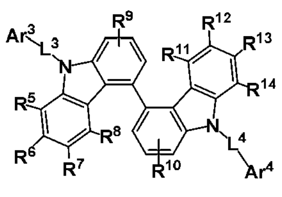
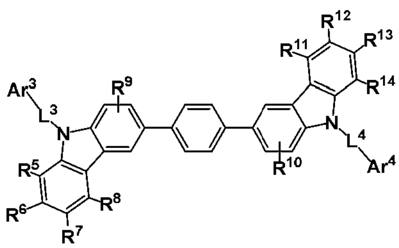
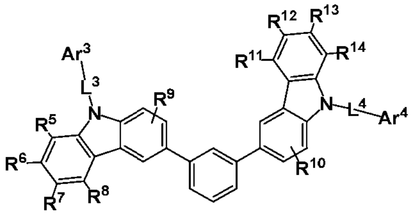

[Chemical Formula 2-13] [Chemical Formula 2-14] [Chemical Formula 2-15]

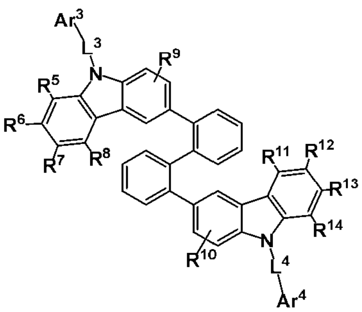 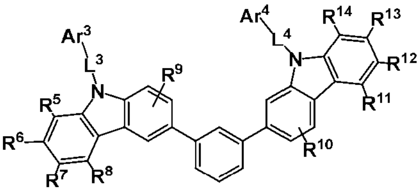 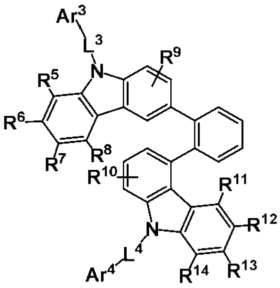

In Chemical Formula 2-1 to Chemical Formula 2-15, $R^5$ to $R^{14}$ may each independently be hydrogen, deuterium or a substituted or unsubstituted C6 to C12 aryl group, and $*-L^3-Ar^3$ and $*-L^4-Ar^4$ may each independently be one of the substituents listed in Group II.

[Group Ⅱ]

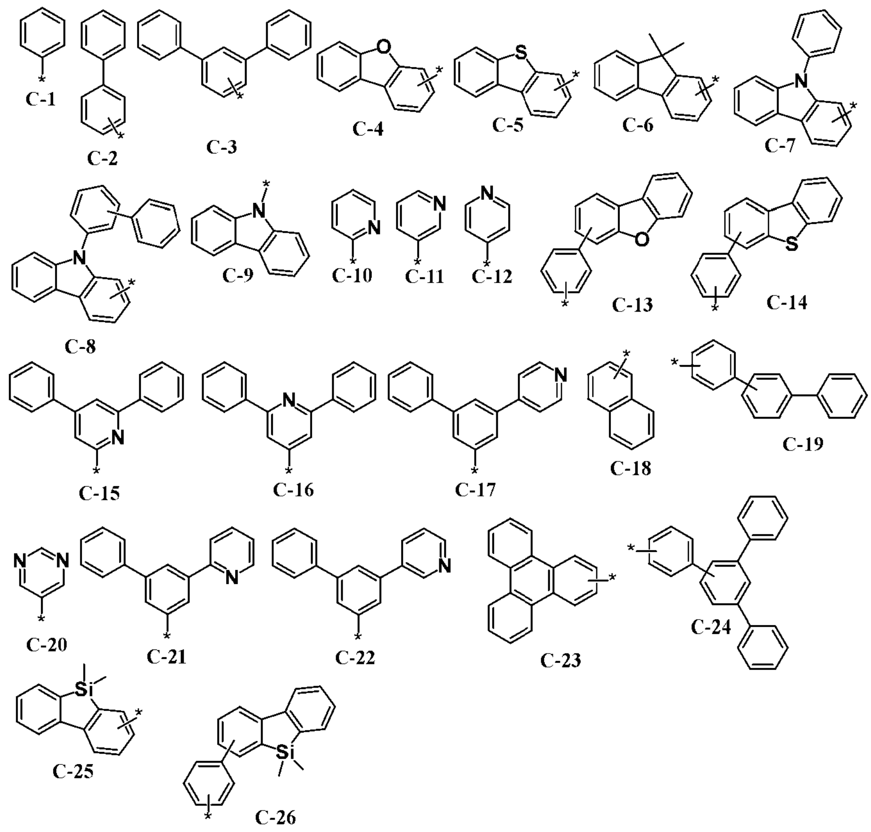

C-1 C-2 C-3 C-4 C-5 C-6 C-7 C-8 C-9 C-10 C-11 C-12 C-13 C-14 C-15 C-16 C-17 C-18 C-19 C-20 C-21 C-22 C-23 C-24 C-25 C-26

In Group II, * is a linking point.

In an embodiment, Chemical Formula 2 may be represented by Chemical Formula 2-8.

In addition, $*-L^3-Ar^3$ and $*-L^4-Ar^4$ of Chemical Formula 2-8 may each independently be selected from Group II, and may be for example any one of C-1, C-2, C-3, C-4, C-7, C-8, and C-9.

As an example, the second compound represented by the combination of Chemical Formula 3 and Chemical Formula 4 may be represented by any one of Chemical Formula 3A, Chemical Formula 3B, Chemical Formula 3C, Chemical Formula 3D, and Chemical Formula 3E.

[Chemical Formula 3A] [Chemical Formula 3B] [Chemical Formula 3C]

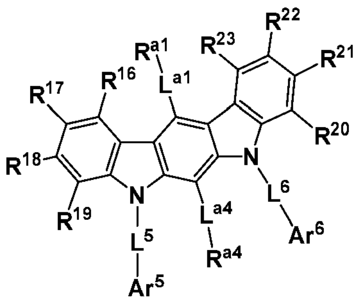

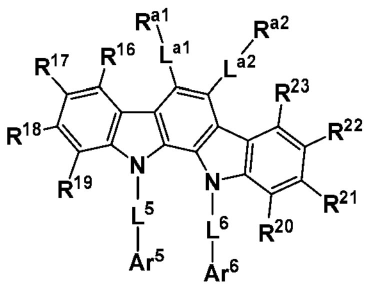

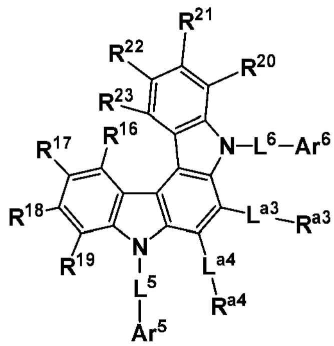

[Chemical Formula 3D] [Chemical Formula 3E]

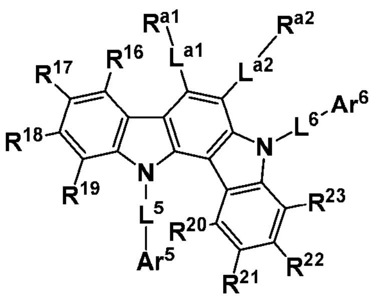

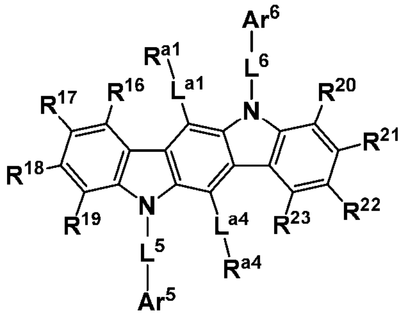

In Chemical Formula 3A to Chemical Formula 3E, $Ar^5$, $Ar^6$, $L^3$, $L^4$, and $R^{16}$ to $R^{23}$ are the same as described above,

$L^{a1}$ to $L^{a4}$ are the same as the definitions of $L^5$ and $L^6$ described above, and
$R^{a1}$ to $R^{a4}$ are the same as the definitions of $R^{16}$ to $R^{23}$ described above.

For example, in Chemical Formulas 3 and 4, $Ar^5$ and $Ar^6$ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, and
$R^{a1}$ to $R^{a4}$ and $R^{16}$ to $R^{23}$ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, $Ar^5$ and $Ar^6$ of Chemical Formulas 3 and 4 may each independently be selected from the substituents listed in Group III.
in an embodiment, the $R^{a1}$ to $R^{a4}$ and $R^{16}$ to $R^{23}$ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, $R^{a1}$ to $R^{a4}$ and $R^{16}$ to $R^{23}$ may each independently be hydrogen, deuterium, a cyano group, or a substituted or unsubstituted phenyl group, and
in a specific embodiment, $R^{a1}$ to $R^{a4}$, and $R^{16}$ to $R^{23}$ may each independently be hydrogen, or a phenyl group.

In a specific embodiment of the present invention, the second compound may be represented by Chemical Formula 2-8, wherein in Chemical Formula 2-8, $Ar^3$ and $Ar^4$ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, $L^3$ and $L^4$ may each independently be a single bond, or a substituted or unsubstituted C6 to C20 arylene group, and $R^5$ to $R^{14}$ may each independently be hydrogen, deuterium, a cyano group, a substituted or

unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific embodiment of the present invention, the second compound may be represented by Chemical Formula 3C, wherein in Chemical Formula 3C, $L^{a3}$ and $L^{a4}$ may be a single bond, $L^5$ and $L^6$ may each independently be a single bond or a substituted or unsubstituted C6 to C12 arylene group, $R^{16}$ to $R^{23}$, $R^{a3}$ and $R^{a4}$ may each be hydrogen or a phenyl group, and $Ar^5$ and $Ar^6$ may each independently be a substituted or unsubstituted phenyl group, a substituted or unsubstituted biphenyl group, or a substituted or unsubstituted terphenyl group.

For example, the second compound may be one selected from the compounds listed in Group 2, but is not limited thereto.

[Group 2]

[B-1] [B-2] [B-3] [B-4] [B-5]

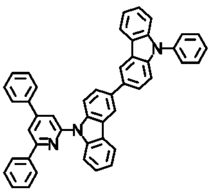
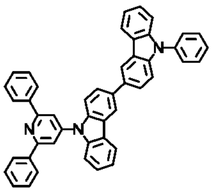
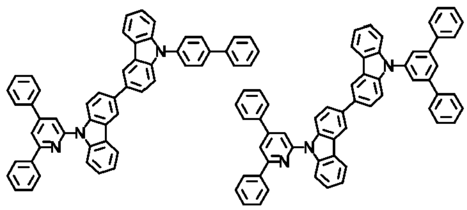
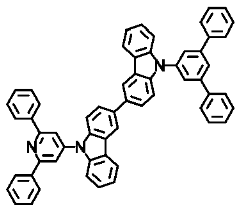

[B-6] [B-7] [B-8] [B-9] [B-10]

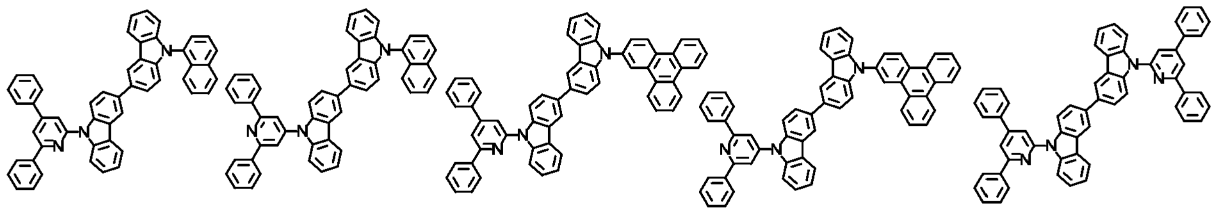

[B-11] [B-12] [B-13] [B-14] [B-15]

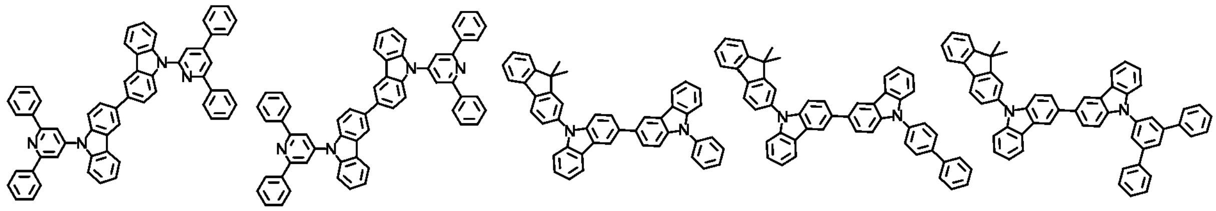

[B-16] [B-17] [B-18] [B-19] [B-20]

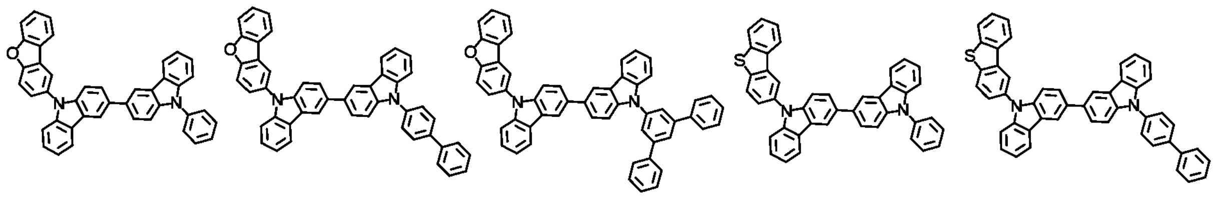

[B-21] [B-22] [B-23] [B-24] [B-25]

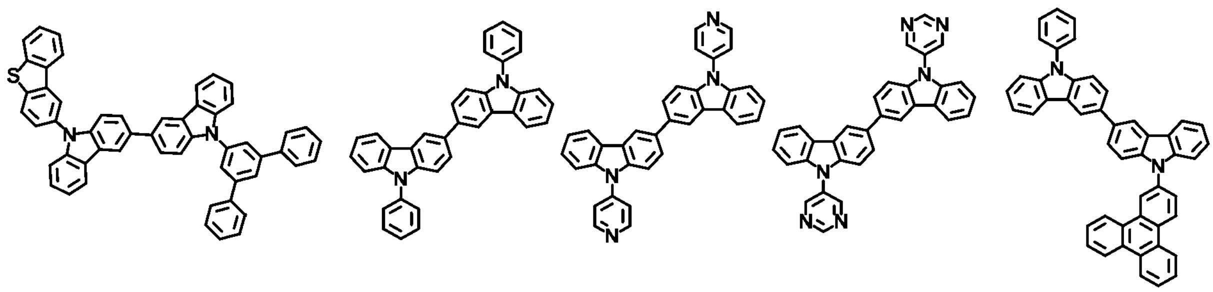

[B-26] [B-27] [B-28] [B-29] [B-30]

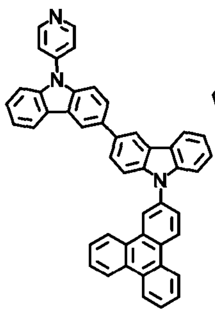 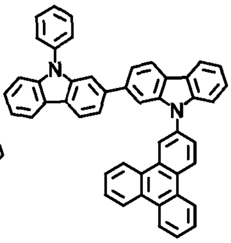 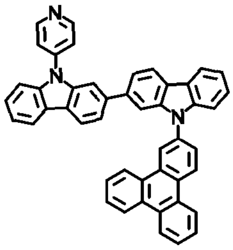 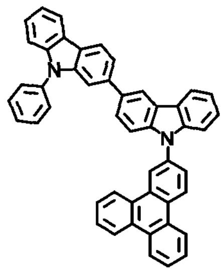 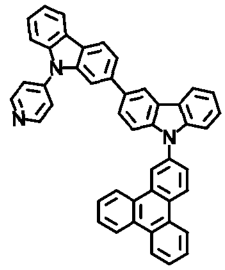

[B-31] [B-32] [B-33] [B-34] [B-35]

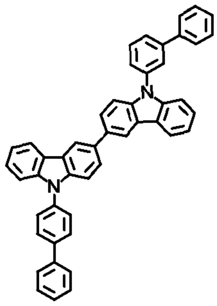 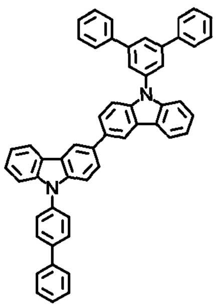 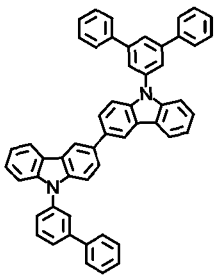 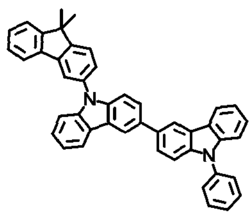 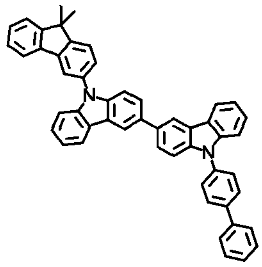

[B-36] [B-37] [B-38] [B-39] [B-40]

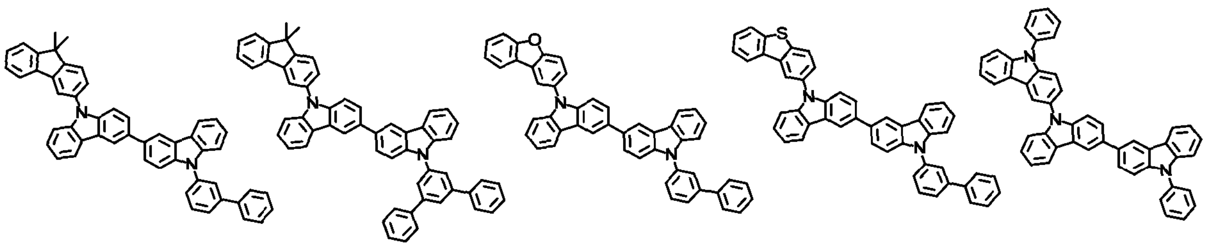

[B-41] [B-42] [B-43] [B-44] [B-45]

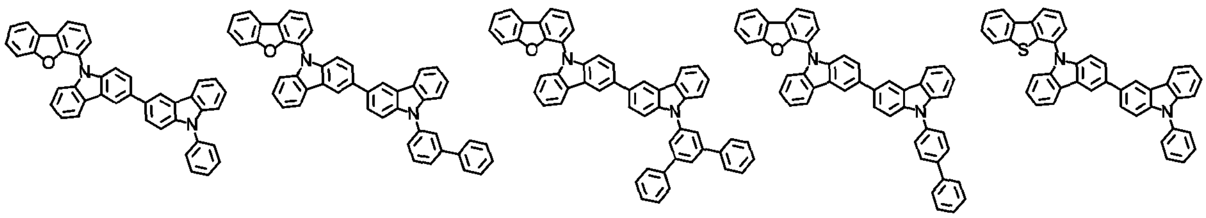

[B-46] [B-47] [B-48] [B-49] [B-50]

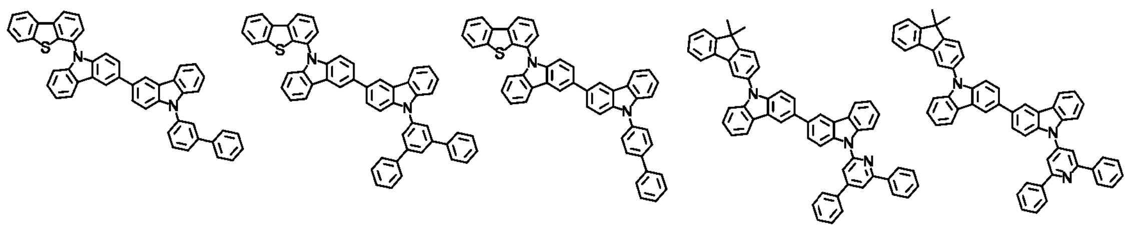

[B-51] [B-52] [B-53] [B-54] [B-55]

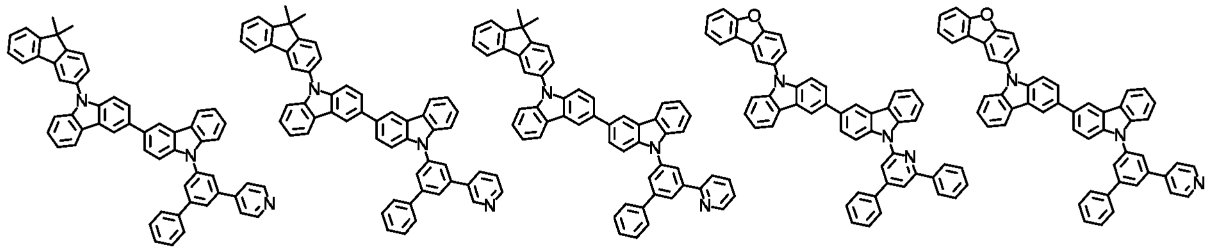

[B-56] [B-57] [B-58] [B-59] [B-60]

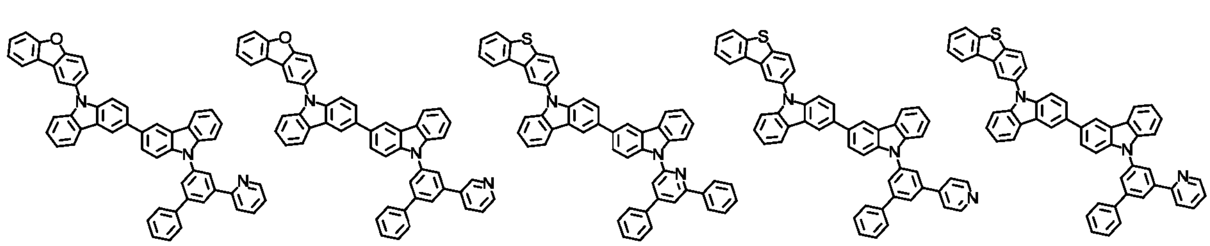

[B-61] [B-62] [B-63] [B-64] [B-65]

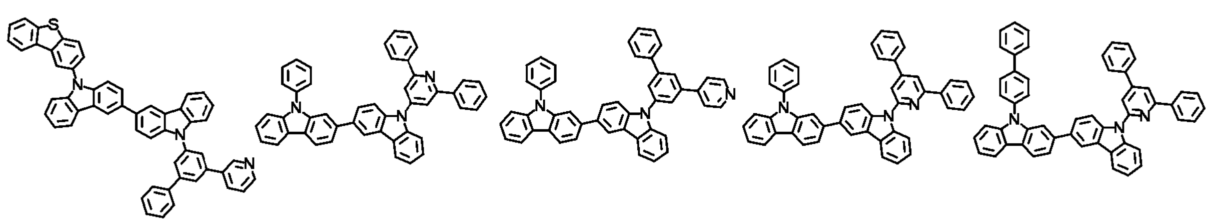

[B-66] [B-67] [B-68] [B-69] [B-70]

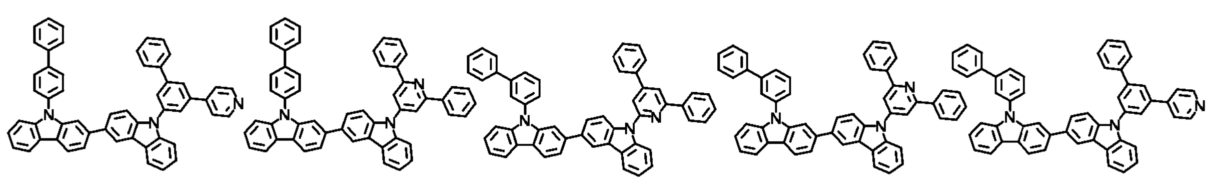

[B-71] [B-72] [B-73] [B-74] [B-75]

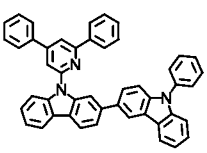 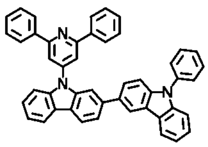 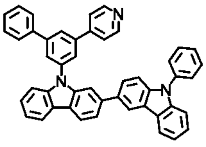 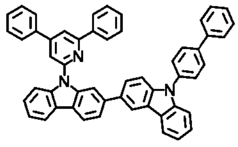 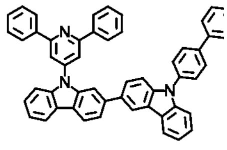

[B-76] [B-77] [B-78] [B-79] [B-80]

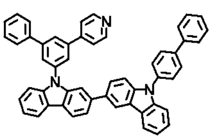 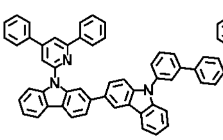 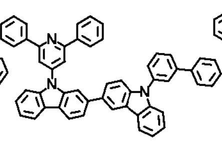 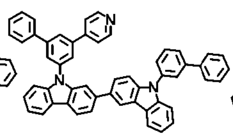 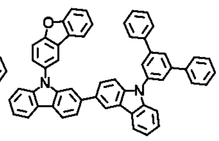

[B-81] [B-82] [B-83] [B-84] [B-85]

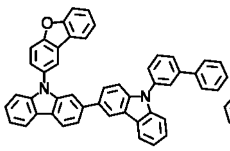 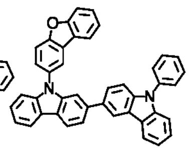 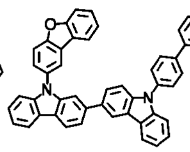 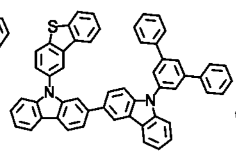 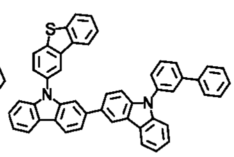

[B-86] [B-87] [B-88] [B-89] [B-90]

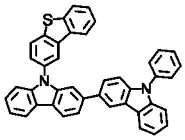 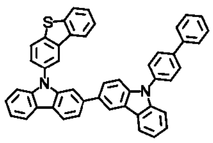 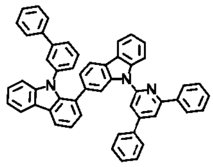 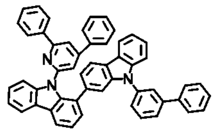 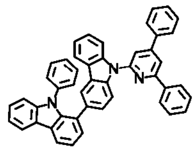

[B-91] [B-92] [B-93] [B-94] [B-95]

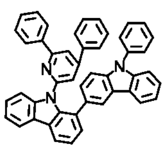 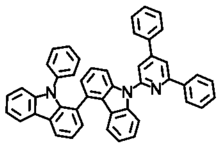 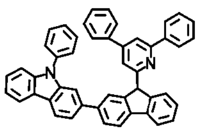 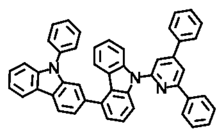 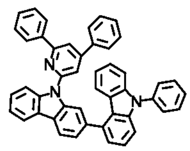

[B-96] [B-97] [B-98] [B-99] [B-100]

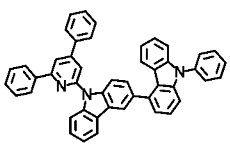 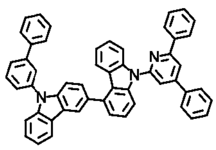 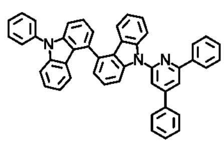 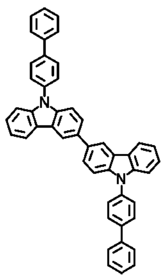 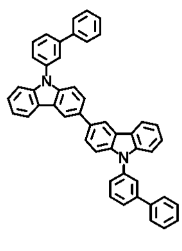

[B-101] [B-102] [B-103] [B-104] [B-105]

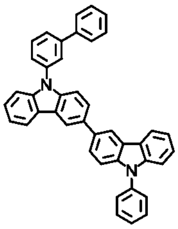
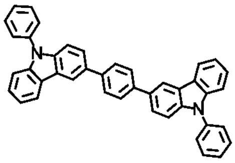
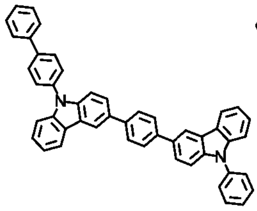
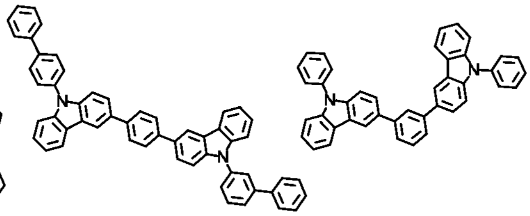

[B-106] [B-107] [B-108] [B-109] [B-110]

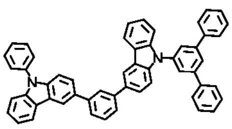
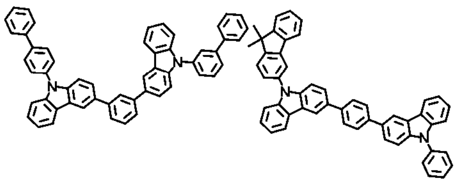
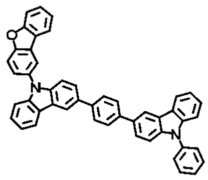
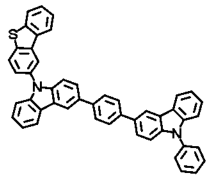

[B-111] [B-112] [B-113] [B-114] [B-115]

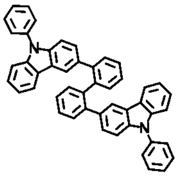
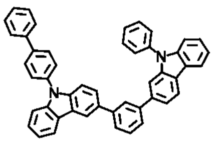
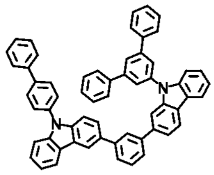
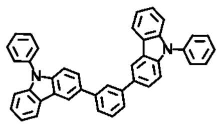
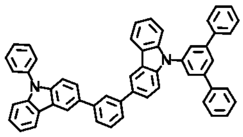

[B-116] [B-117] [B-118] [B-119] [B-120]

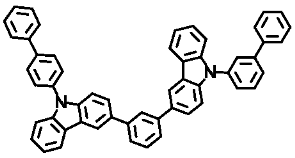
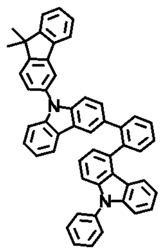
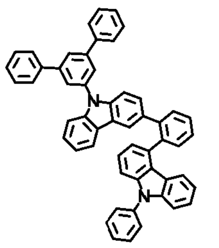
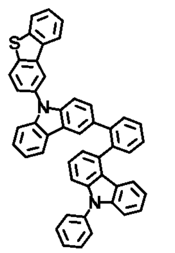
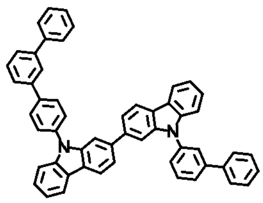

[B-121] [B-122] [B-123] [B-124] [B-125]

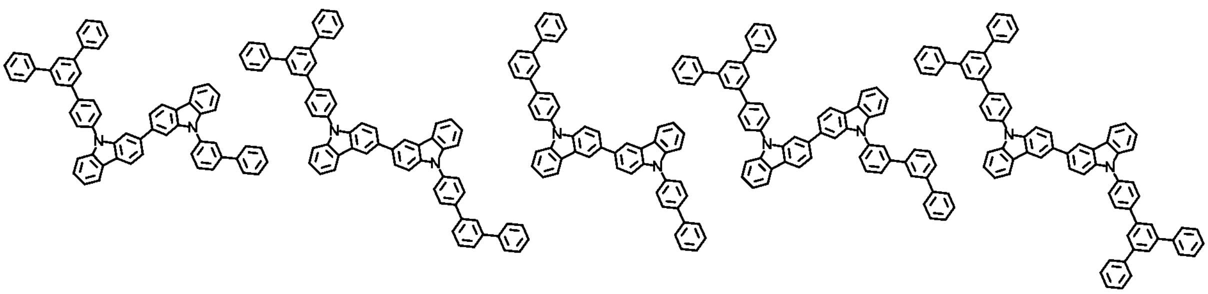

[B-126] [B-127] [B-128] [B-129] [B-130]

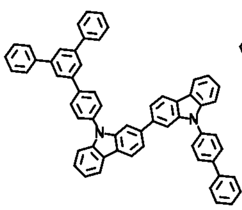
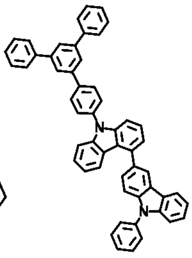
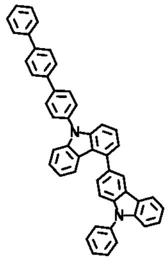
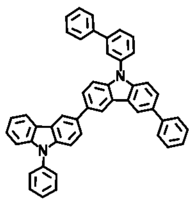
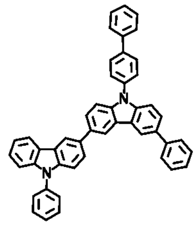

[B-131] [B-132] [B-133] [B-134] [B-135]

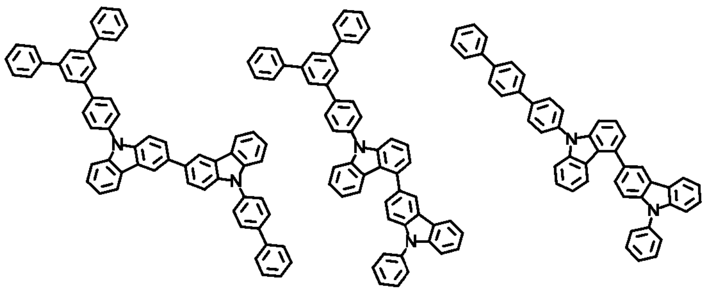
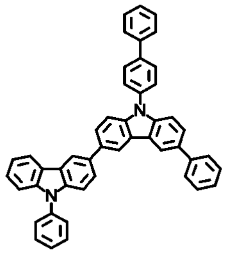
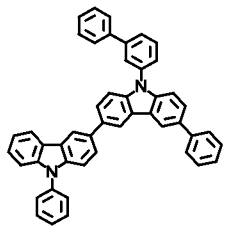

[B-136] [B-137] [B-138]

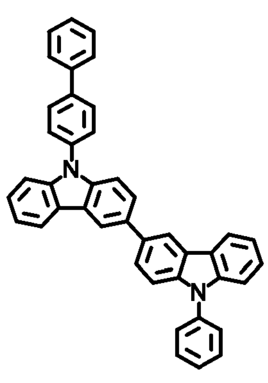
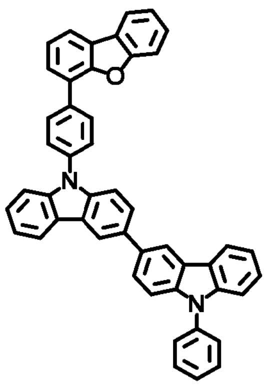
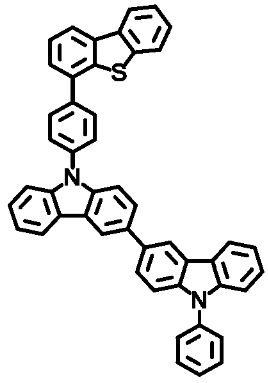

[B-139] [B-140] [B-141] [B-142]

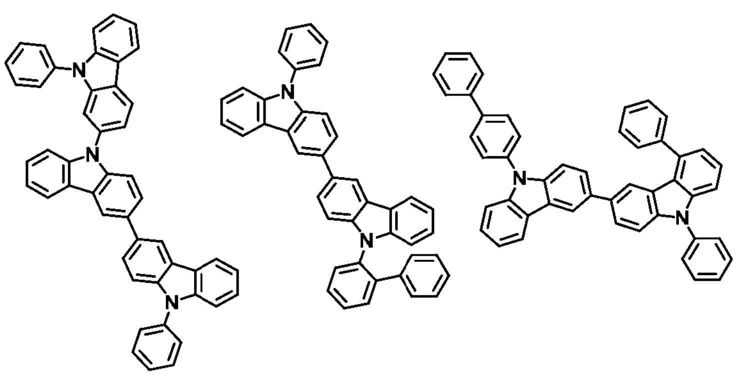
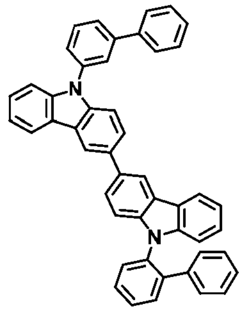

[B-143] [B-144] [B-145] [B-146]

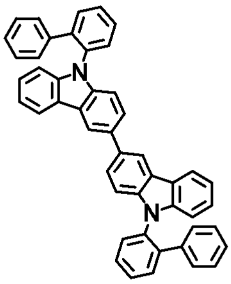 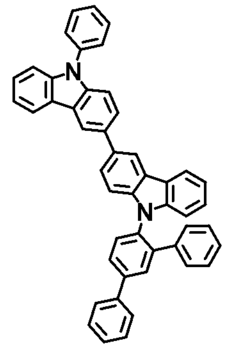 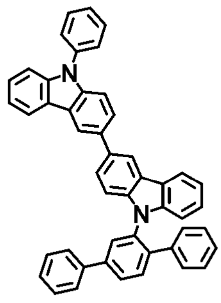 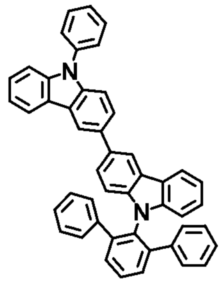

[B-147] [B-148] [B-149] [B-150]

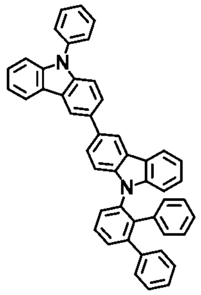 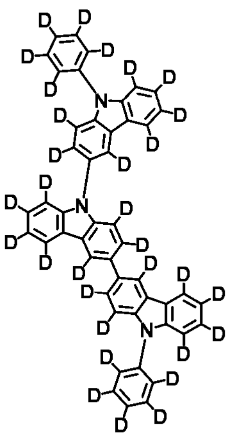 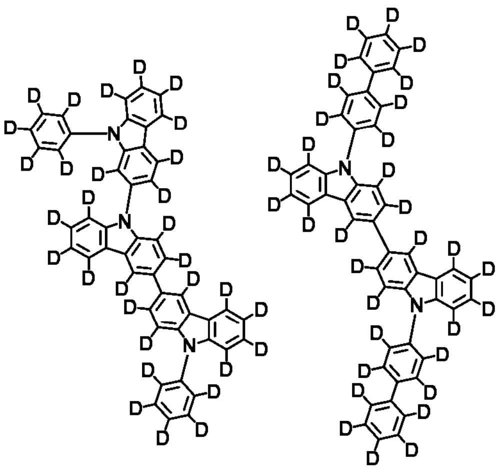

[B-151] [B-152] [B-153] [B-154]

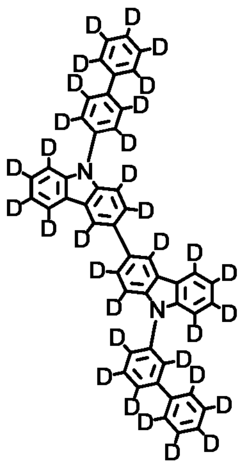 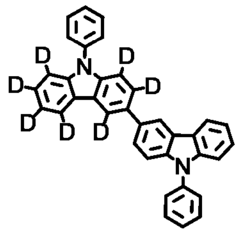 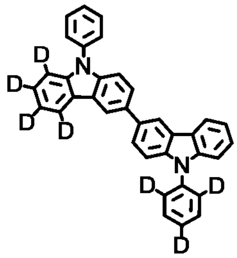 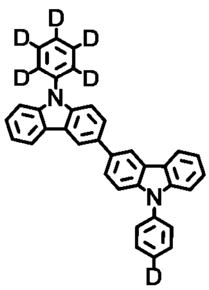

[B-155] [B-156] [B-157] [B-158]

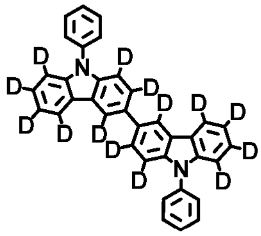

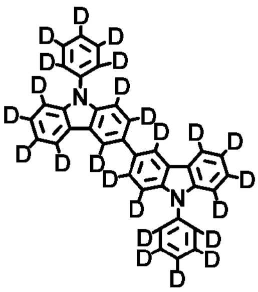

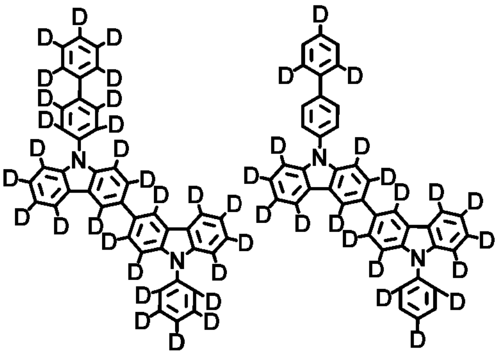

[B-159] [B-160] [B-161] [B-162]

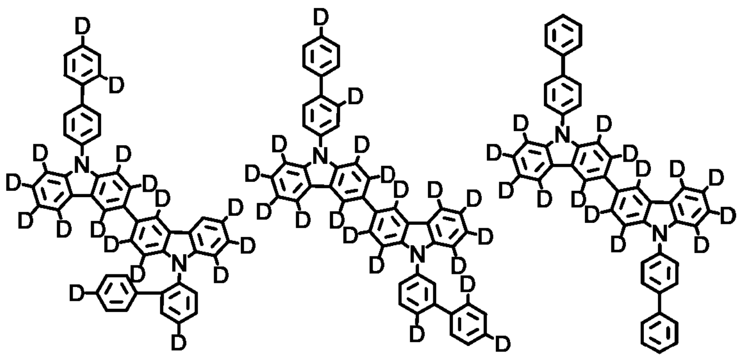

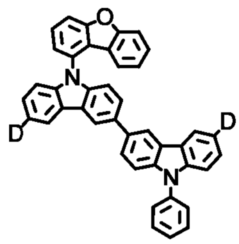

[B-163] [B-164] [B-165] [B-166]

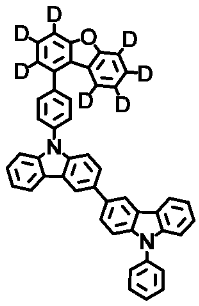

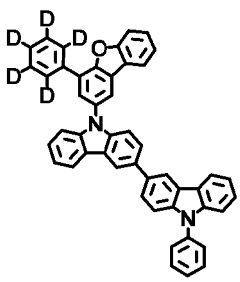

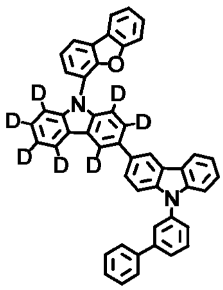

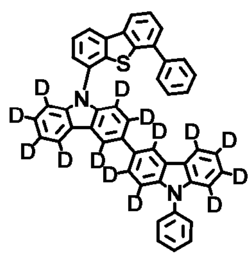

[B-167] [B-168] [B-169]

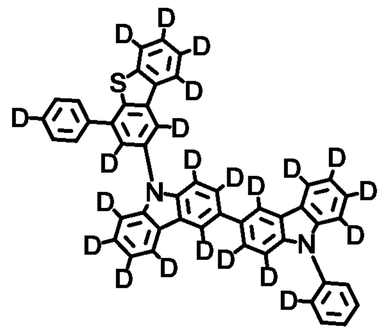
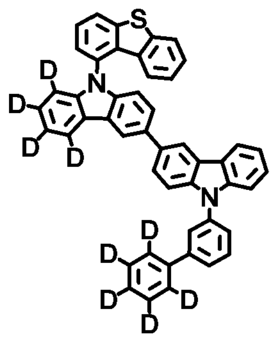
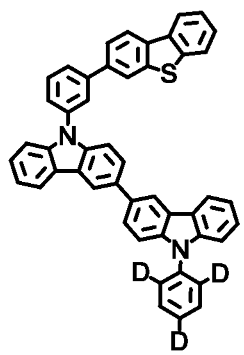

[B-170] [B-171] [B-172] [B-173]

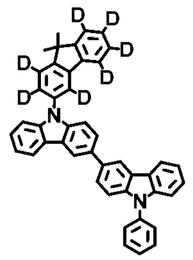
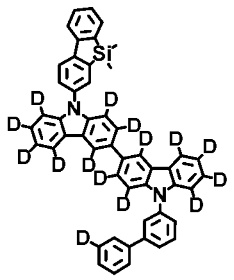
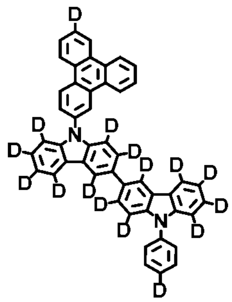
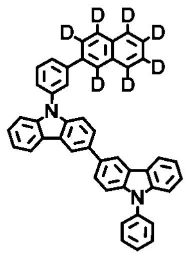

[B-174] [B-175] [B-176] [B-177]

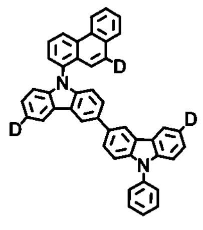
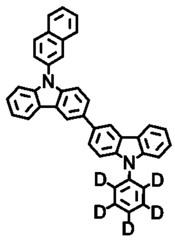
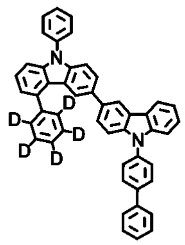
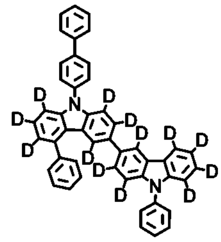

[B-178] [B-179] [B-180] [B-181]

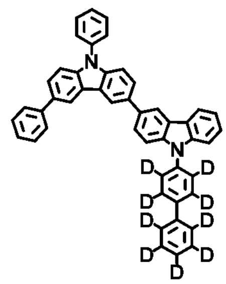
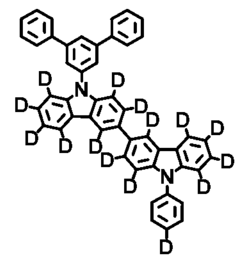
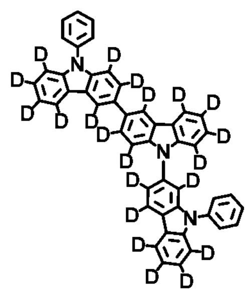
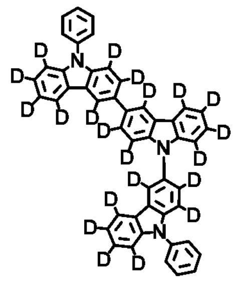

[B-182] [B-183] [B-184] [B-185]

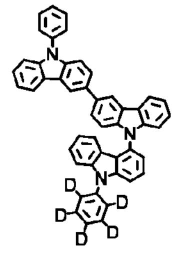 [C-1]

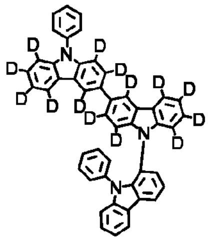 [C-2]

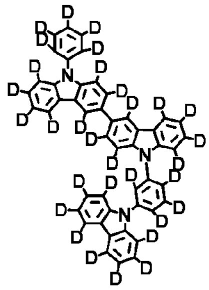 [C-3]

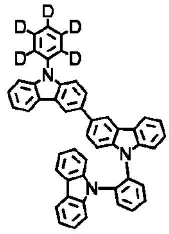 [C-4]

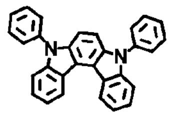 [C-5]

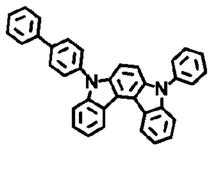 [C-6]

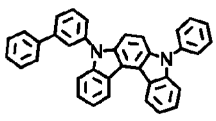 [C-7]

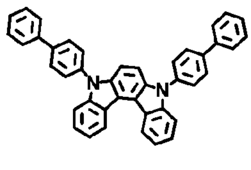 [C-8]

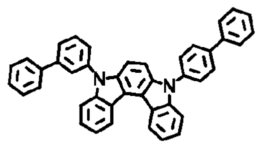 [C-9]

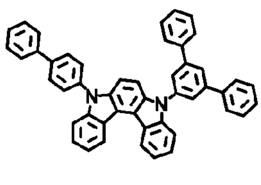 [C-10]

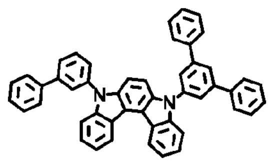 [C-11]

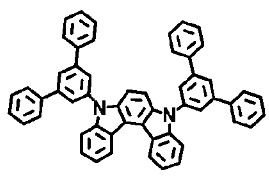 [C-12]

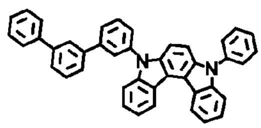 [C-13]

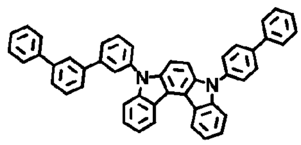 [C-14]

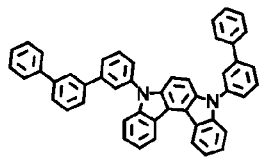 [C-15]

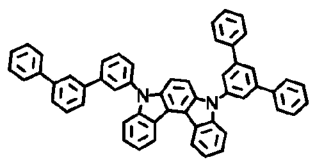 [C-16]

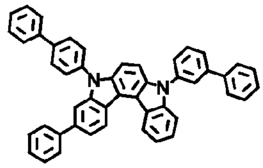 [C-17]

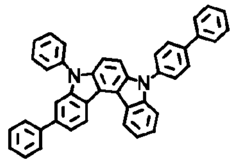 [C-18]

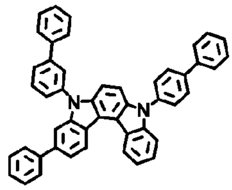 [C-19]

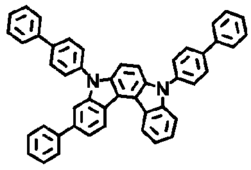 [C-20]

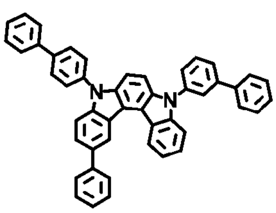
[C-21]

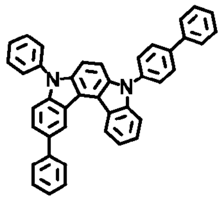
[C-22]

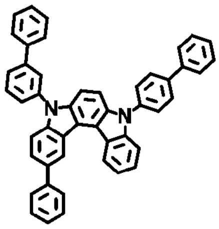
[C-23]

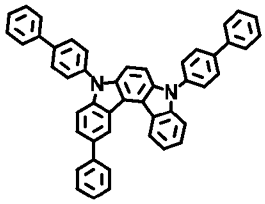
[C-24]

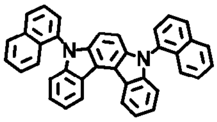
[C-25]

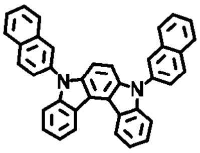
[C-26]

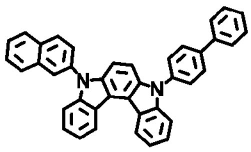
[C-27]

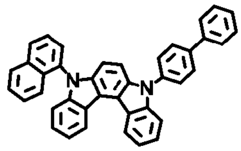
[C-28]

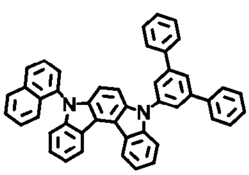
[C-29]

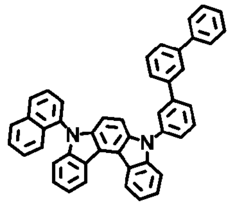
[C-30]

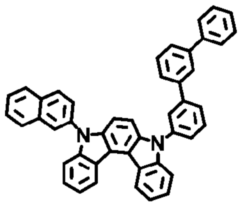
[C-31]

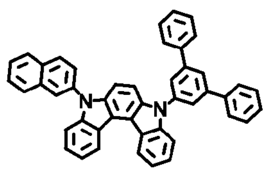
[C-32]

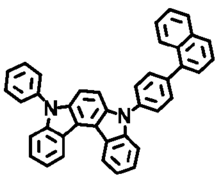
[C-33]

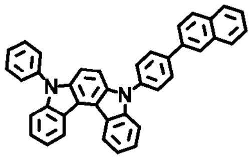
[C-34]

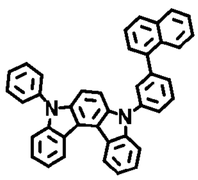
[C-35]

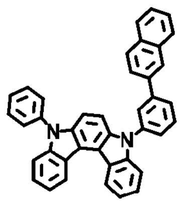
[C-36]

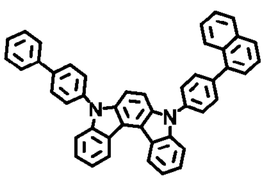
[C-37]

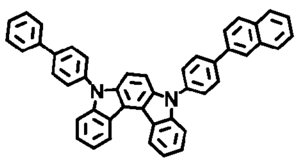
[C-38]

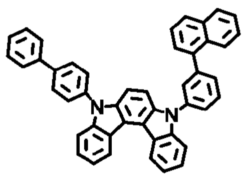
[C-39]

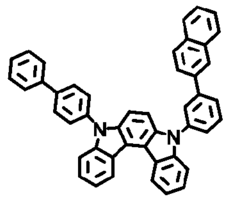
[C-40]

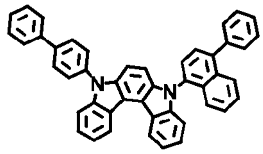 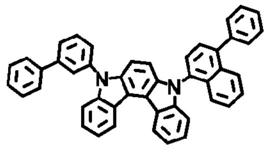 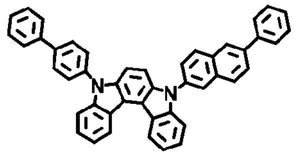 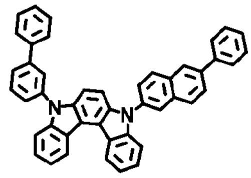

[C-41] [C-42] [C-43] [C-44]

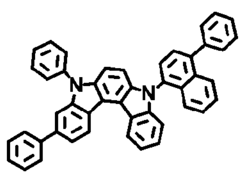 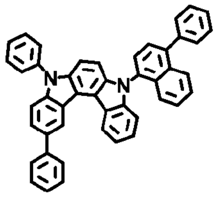 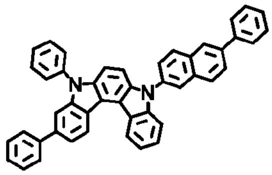 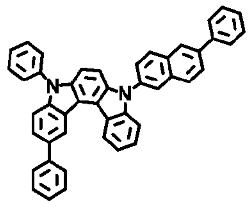

[C-45] [C-46] [C-47] [C-48]

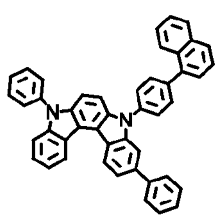 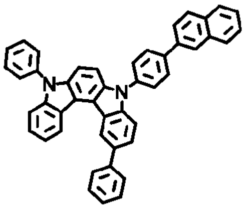 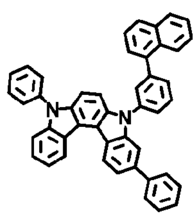 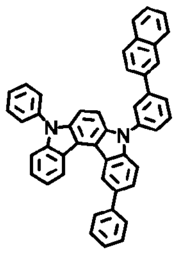

[C-49] [C-50] [C-51] [C-52]

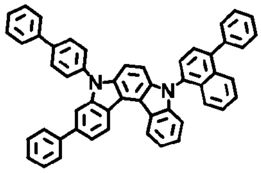 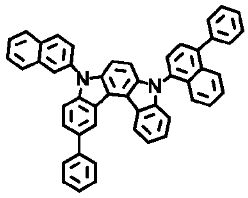 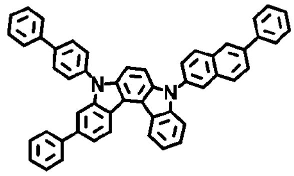 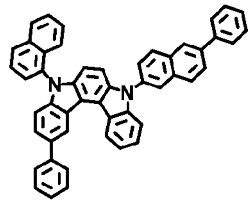

[C-53] [C-54] [C-55] [C-56]

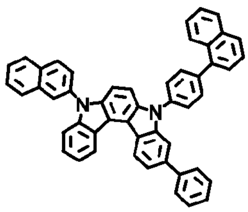 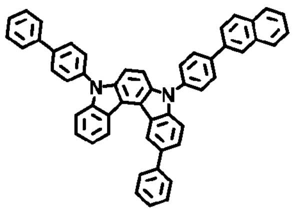 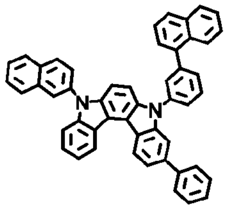 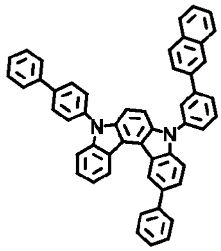

[C-57] [C-58] [C-59]

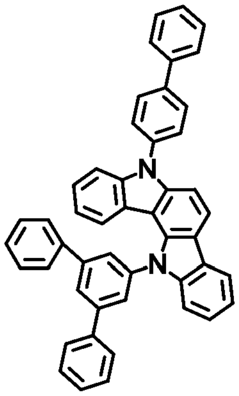
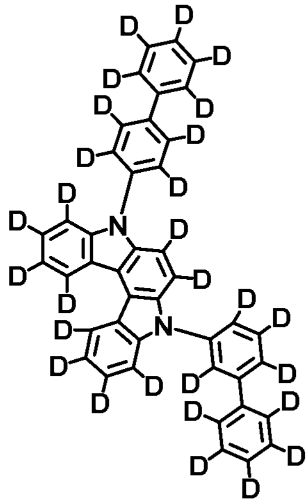
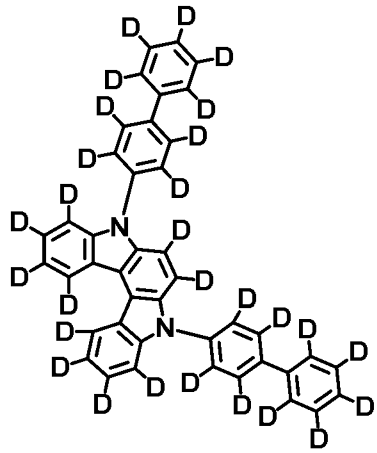

[C-60] [C-61] [C-62] [C-63]

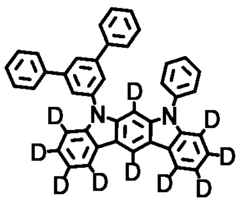
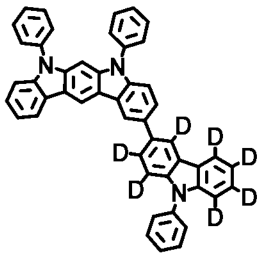
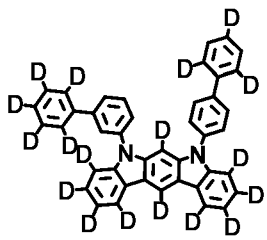
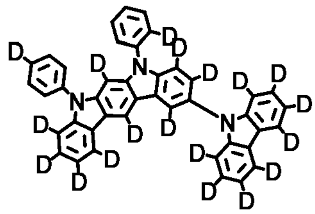

[C-64] [C-65] [C-66] [C-67]

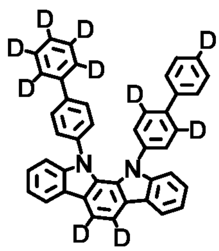
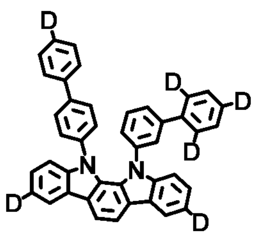
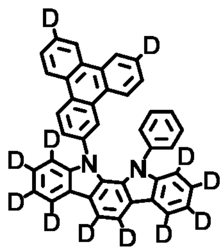
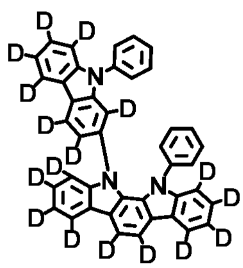

[C-68] [C-69] [C-70] [C-71]

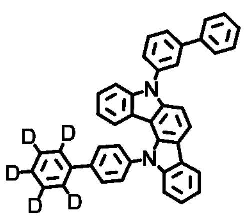
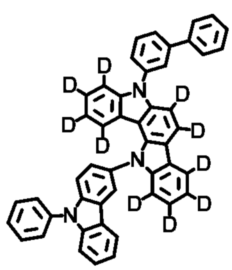
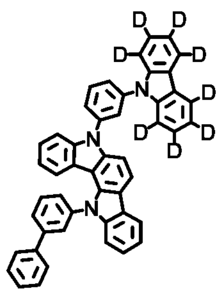
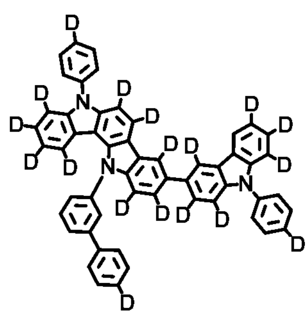

[C-72]

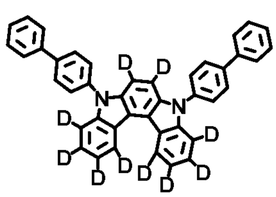

[C-73]

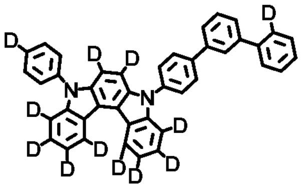

[C-74]

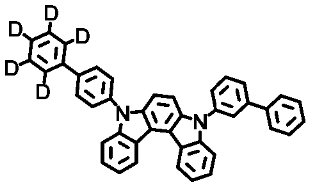

[C-75]

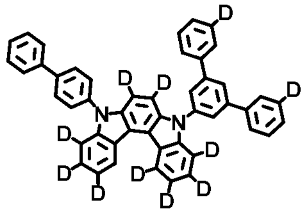

[C-76]

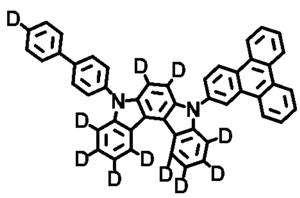

[C-77]

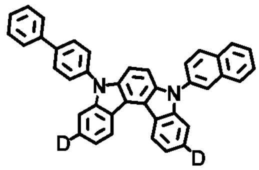

[C-78]

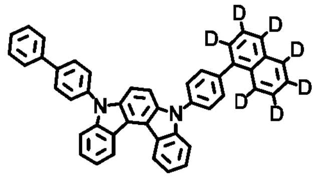

[C-79]

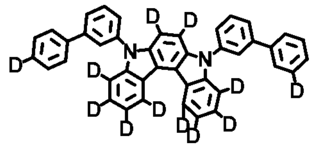

[C-80]

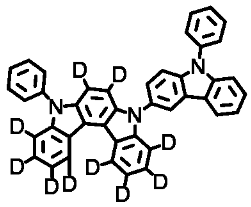

[C-81]

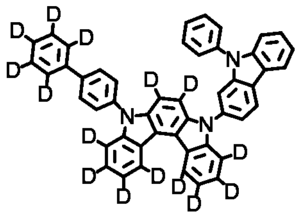

[C-82]

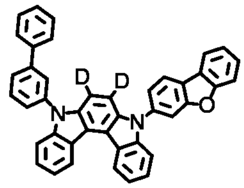

[C-83]

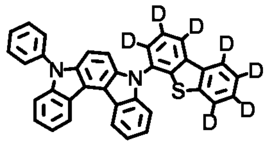

[C-84]

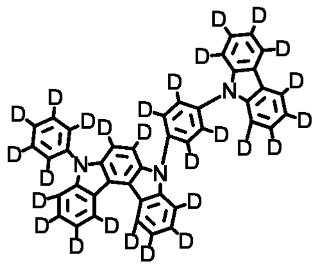

[C-85]

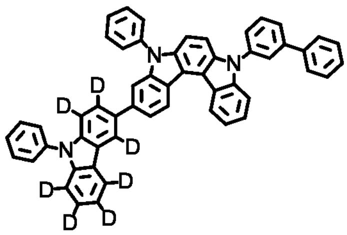

[C-86]

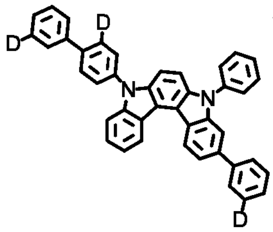

[C-87]

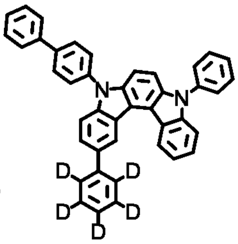

The first compound and the second compound may be included in a weight ratio of, for example, 1:99 to 99:1. Within the above range, efficiency and life-span can be improved by implementing bipolar characteristics by adjusting the appropriate weight ratio using the electron transport ability of the first compound and the hole transport ability of the second compound. Within the above range, they may be included in a weight ratio of, for example, about 10:90 to 90:10, about 20:80 to 80:20, for example, about 20:80 to about 70:30, about 20:80 to about 60:40, or about 20:80 to about 50:50. As a specific example, they may be included in a weight ratio of 20:80, 30:70, or 40:60.

In addition to the aforementioned first and second compounds, one or more compounds may be further included.

For example, the aforementioned compound for an organic optoelectronic device or composition for an organic optoelectronic device may further include a dopant.

The dopant may be, for example, a phosphorescent dopant, such as a red, green, or blue phosphorescent dopant, and may be, for example, a red or green phosphorescent dopant.

The dopant is a material mixed with the compound for an organic optoelectronic device in a small amount to cause light emission and may be generally a material such as a metal complex that emits light by multiple excitation into a triplet or more. The dopant may be, for example an inorganic, organic, or organic-inorganic compound, and one or more types

thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] $L^7MX$

In Chemical Formula Z, M is a metal, and $L^7$ and X are the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof, and $L^6$ and X may be, for example a bidentate ligand.

Examples of the ligands represented by $L^7$ and X may be selected from the chemical formulas listed in Group A, but are not limited thereto.

[Group A]

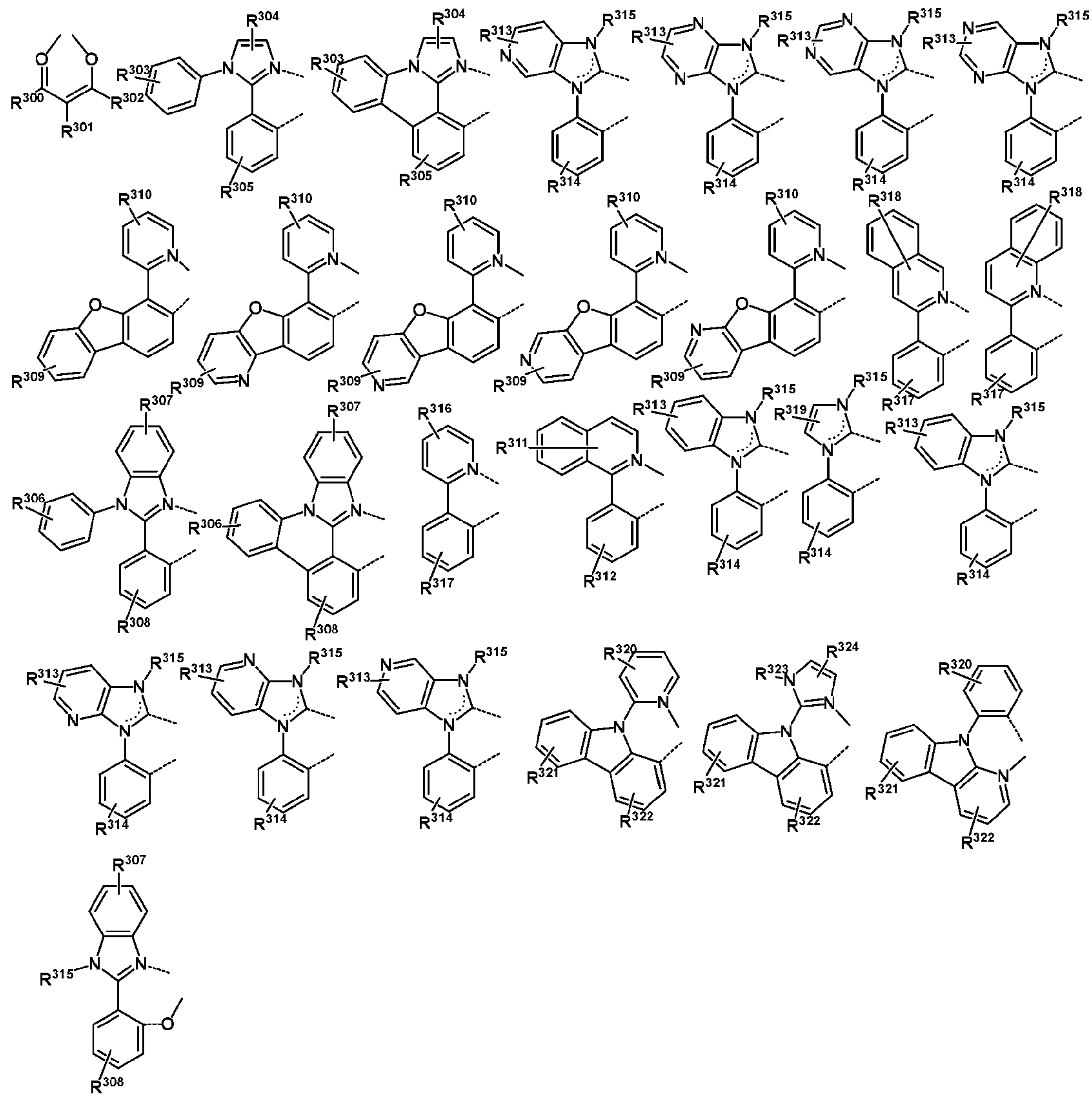

In Group A,

$R^{300}$ to $R^{302}$ are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and

$R^{303}$ to $R^{324}$ are each independently hydrogen, deuterium, a halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, $SF_5$, a trialkylsilyl group having a substituted or unsubstituted C1 to C30 alkyl group, a dialkylarylsilyl group having a substituted or unsubstituted C1 to C30 alkyl group and C6 to C30 aryl group, or a triarylsilyl group having a substituted or unsubstituted C6 to C30 aryl group.

For example, a dopant represented by Chemical Formula V may be included.

[Chemical Formula V]

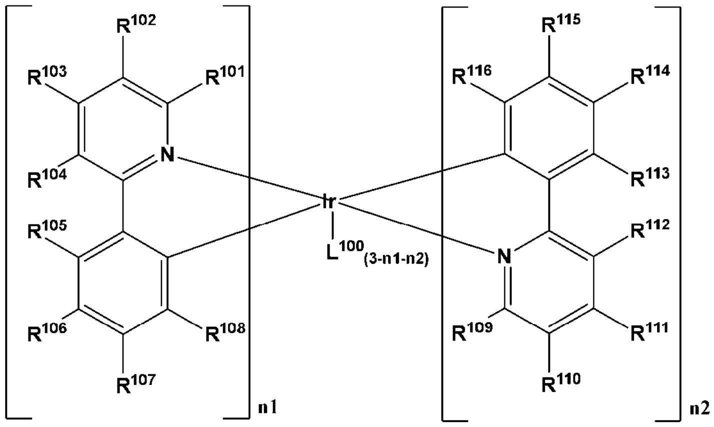

In Chemical Formula V,

$R^{101}$ to $R^{116}$ are each independently hydrogen, deuterium, a substituted or unsubstituted C1 to C10 alkyl group, a substituted or unsubstituted C6 to C20 aryl group, or $-SiR^{132}R^{133}R^{134}$,
$R^{132}$ to $R^{134}$ are each independently a C1 to C6 alkyl group,
at least one of $R^{101}$ to $R^{116}$ is a functional group represented by Chemical Formula V-1,
$L^{100}$ is a bidentate ligand of a monovalent anion, and is a ligand that coordinates to iridium through a lone pair of carbons or heteroatoms, and
n1 and n2 are each independently any one of integers of 0 to 3, and n1 + n2 is any one of integers of 1 to 3,

[Chemical Formula V-1]

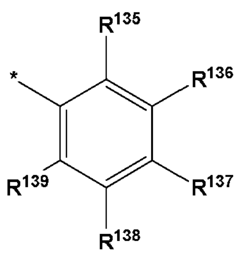

wherein, in Chemical Formula V-1,
$R^{135}$ to $R^{139}$ are each independently hydrogen, deuterium, a substituted or unsubstituted C1 to C10 alkyl group, a substituted or unsubstituted C6 to C20 aryl group, or $-SiR^{132}R^{133}R^{134}$, and
* means a portion linked to a carbon atom.

For example, the dopant represented by Chemical Formula Z-1 may be included.

[Chemical Formula Z-1]

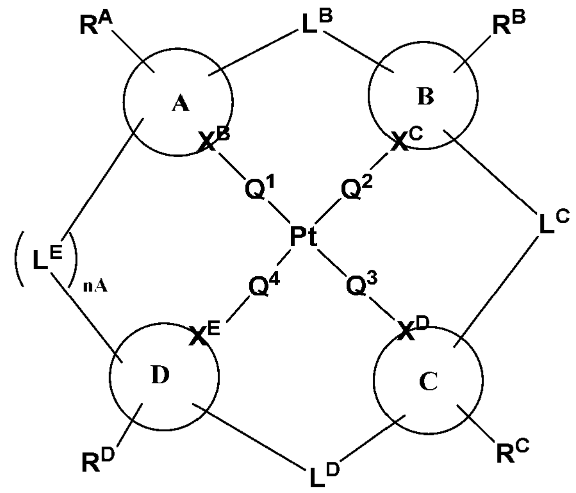

In Chemical Formula Z-1, rings A, B, C, and D are each independently a 5-membered or 6-membered carbocyclic or heterocyclic ring;

$R^A$, $R^B$, $R^C$, and $R^D$ are each independently mono-, di-, tri-, or tetra-substitution, or unsubstitution;
$L^B$, $L^C$, and $L^D$ are each independently a direct bond, BR, NR, PR, O, S, Se, C=O, S=O, $SO_2$, CRR', SiRR', GeRR', or a combination thereof,
when nA is 1, $L^E$ may be a direct bond, BR, NR, PR, O, S, Se, C=O, S=O, $SO_2$, CRR', SiRR', GeRR', or a combination thereof; and when nA is 0, $L^E$ does not exist;
$R^A$, $R^B$, $R^C$, $R^D$, R, and R' are each independently hydrogen, deuterium, a halogen, an alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, or a combination thereof; any adjacent $R^A$, $R^B$, $R^C$, $R^D$, R, and R' are optionally linked to each other to provide a ring; $X^B$, $X^C$, $X^D$, and $X^E$ are each independently selected from carbon and nitrogen; and $Q^1$, $Q^2$, $Q^3$, and $Q^4$ each represent oxygen or a direct bond.

The dopant according to an embodiment may be a platinum complex, and may be represented by Chemical Formula VI.

[Chemical Formula VI]

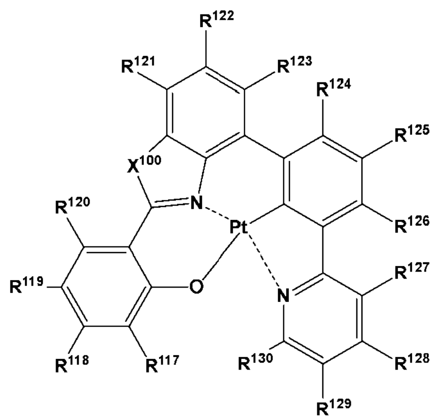

In Chemical Formula VI,

$X^{100}$ is selected from O, S, and $NR^{131}$,
$R^{117}$ to $R^{131}$ are each independently hydrogen, deuterium, a substituted or unsubstituted C1 to C10 alkyl group, a substituted or unsubstituted C6 to C20 aryl group, or - $SiR^{132}R^{133}R^{134}$,
$R^{132}$ to $R^{134}$ are each independently a C1 to C6 alkyl group, and
at least one of $R^{117}$ to $R^{131}$ is $-SiR^{132}R^{133}R^{134}$ or a tert-butyl group.

Hereinafter, an organic optoelectronic device including the aforementioned compound for an organic optoelectronic device or composition for an organic optoelectronic device will be described.
The organic optoelectronic device may be a suitable device to convert electrical energy into photoenergy and vice versa, e.g., an organic photoelectric device, an organic light emitting diode, an organic solar cell, or an organic photoconductor drum.
Herein, an organic light emitting diode as one example of an organic optoelectronic device is described referring to drawings.
FIG. 1 is a cross-sectional view showing an organic light emitting diode according to an embodiment.
Referring to FIG. 1, an organic light emitting diode 100 according to an embodiment includes an anode 120 and a cathode 110 facing each other and an organic layer 105 disposed between the anode 120 and cathode 110.
The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or $SnO_2$ and Sb; a conductive polymer such as poly(3-methylthiophene), poly(3,4-(ethylene-1,2-dioxy)thiophene) (PEDOT), polypyrrole, and polyaniline, but is not limited thereto.
The cathode 110 may be made of a conductor having a small work function to help electron injection, and may be for example a metal, a metal oxide, and/or a conductive polymer. The cathode 110 may be for example a metal such as magnesium, calcium, sodium, potassium, titanium, indium, yttrium, lithium, gadolinium, aluminum silver, tin, lead, cesium, barium, and the like, or an alloy thereof; a multi-layer structure material such as LiF/Al, $LiO_2$/Al, LiF/Ca, and $BaF_2$/Ca, but is not limited thereto.
The organic layer 105 may include the aforementioned compound for an organic optoelectronic device or composition for an organic optoelectronic device.
The organic layer 105 may include a light emitting layer 130 and the light emitting layer 130 may include the aforementioned compound for an organic optoelectronic device or composition for an organic optoelectronic device.
The composition for an organic optoelectronic device further including a dopant may be, for example, a red or green light emitting composition.
The light emitting layer 130 may include, for example, the aforementioned compound for an organic optoelectronic device or composition for an organic optoelectronic device, as a phosphorescent host.
The organic layer may further include a charge transport region in addition to the light emitting layer.
The charge transport region may be, for example, a hole transport region 140.
The hole transport region 140 may further increase hole injection and/or hole mobility between the anode 120 and the light emitting layer 130 and block electrons.
Specifically, the hole transport region 140 may include a hole transport layer between the anode 120 and the light emitting layer 130, and a hole transport auxiliary layer between the light emitting layer 130 and the hole transport layer, and at least one of the compounds of Group B may be included in at least one of the hole transport layer and the hole transport auxiliary layer.

[Group B]

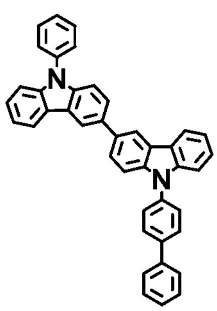
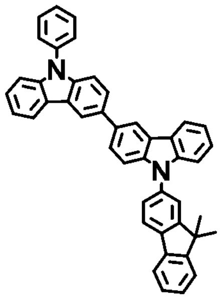
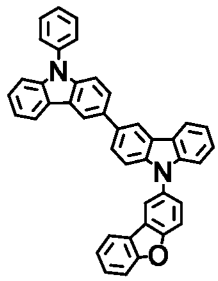
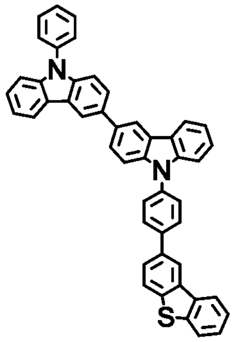

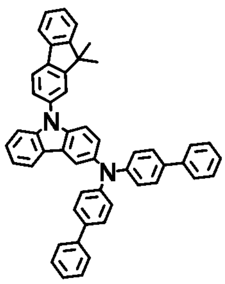
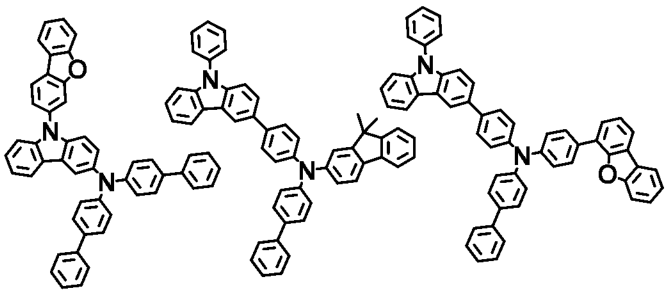

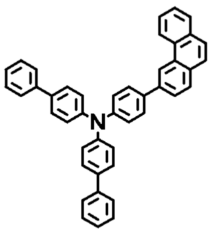
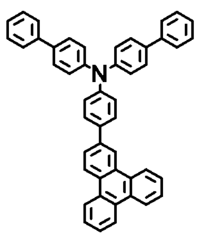
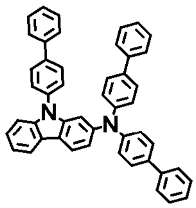
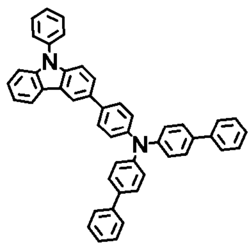

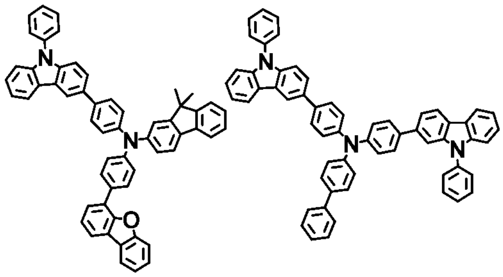
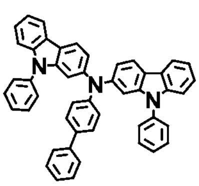
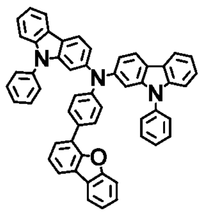

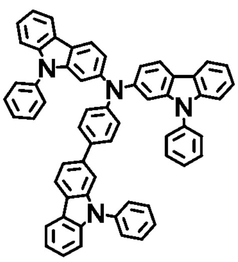

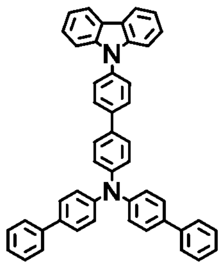

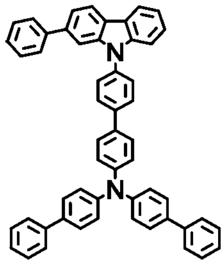

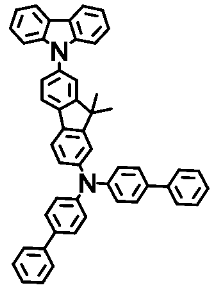

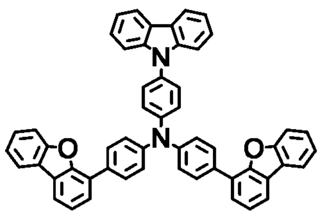

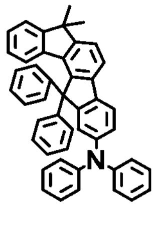

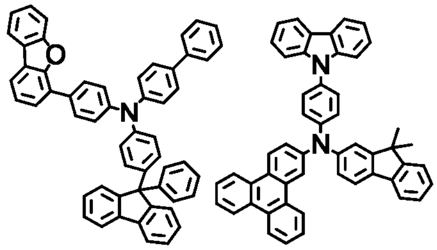

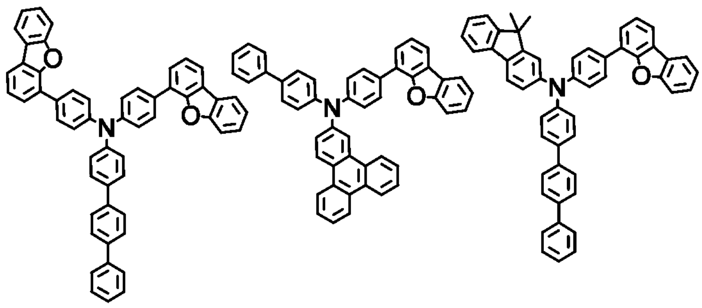

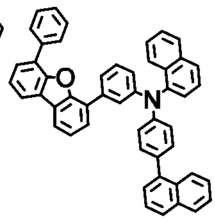

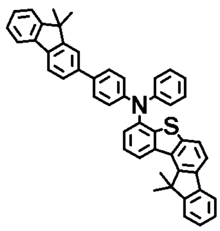

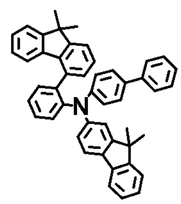

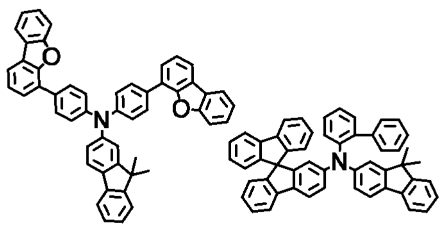

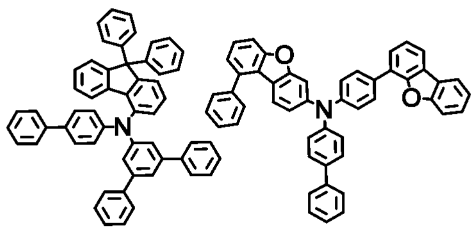

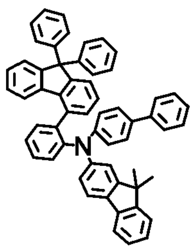

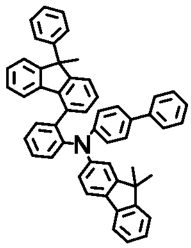

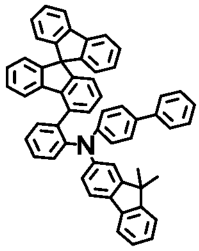
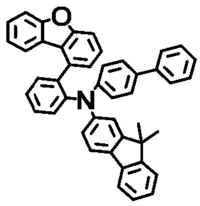
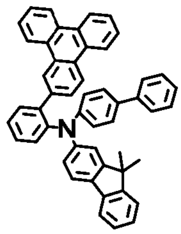
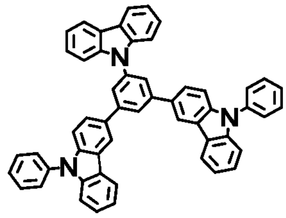
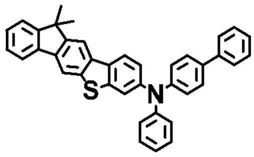
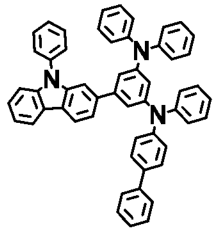
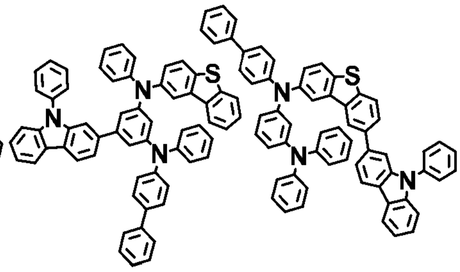
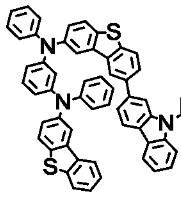
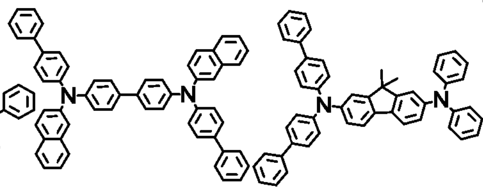
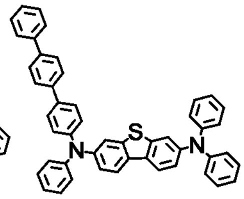
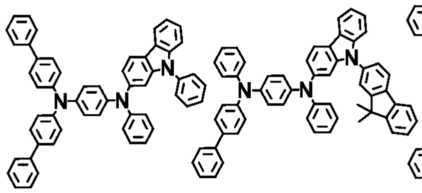
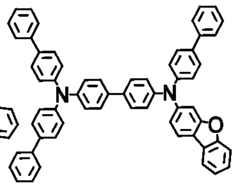
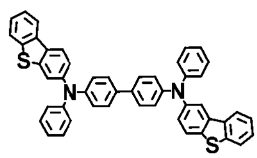
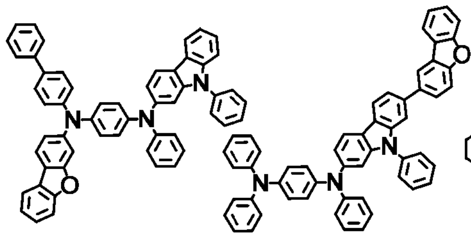
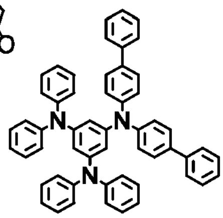
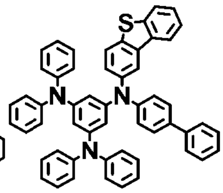
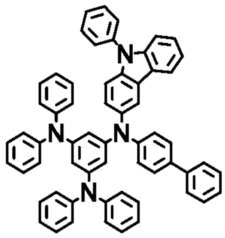
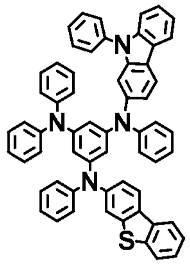
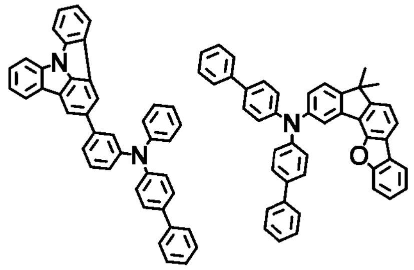

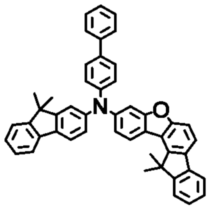
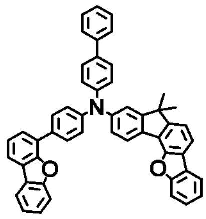
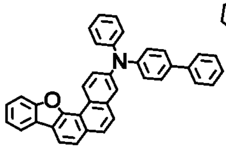
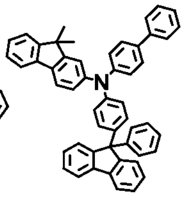

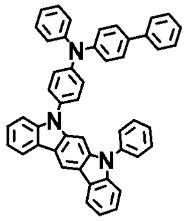
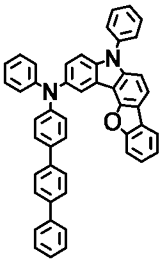
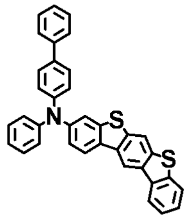
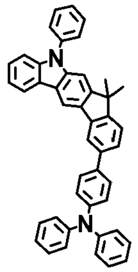

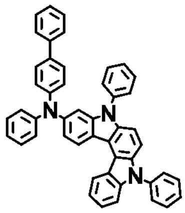
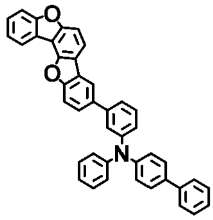

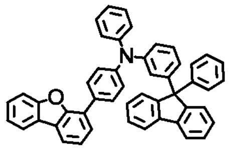
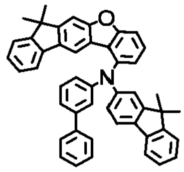
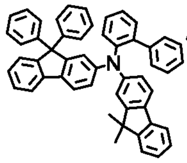
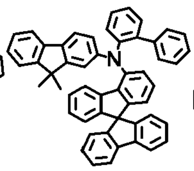
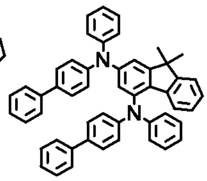

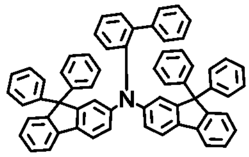
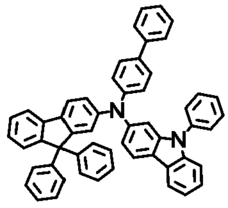
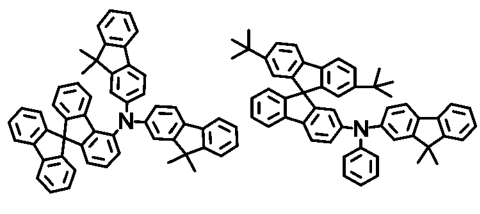

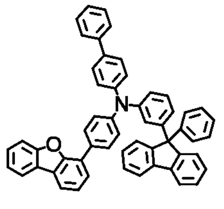
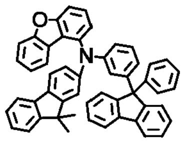
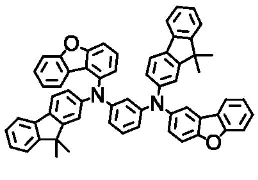
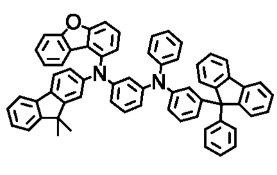

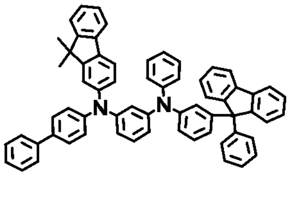
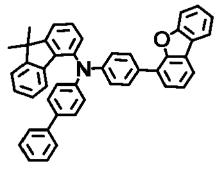
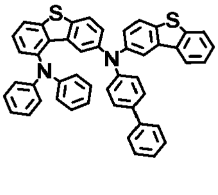
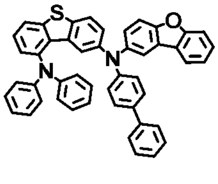

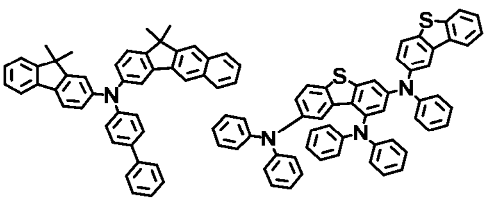
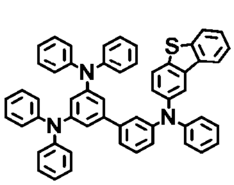
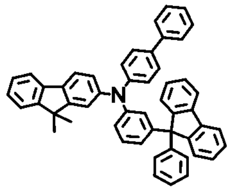

In the hole transport region, in addition to the compounds described above, known compounds disclosed in US5061569A, JP1993-009471A, WO1995-009147A1, JP1995-126615A, JP1998-095973A, etc. and compounds having a similar structure may also be used.

Also, the charge transport region may be, for example, the electron transport region 150.

The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group C may be included in at least one of the electron transport layer and the electron transport auxiliary layer.

[Group C]

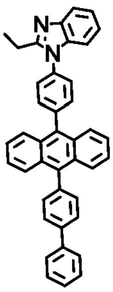
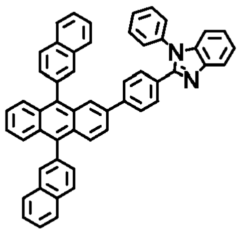
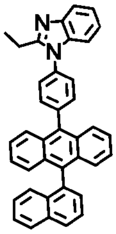
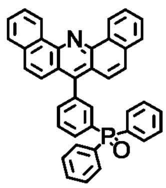
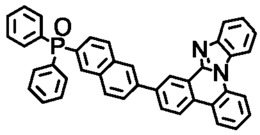

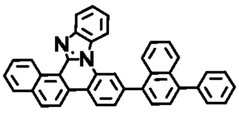
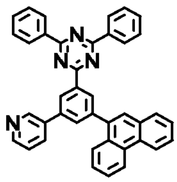
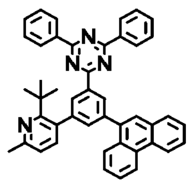
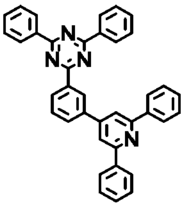
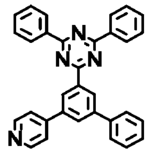

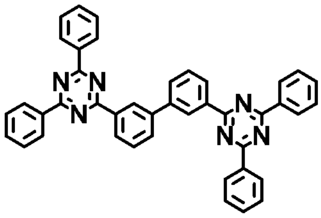
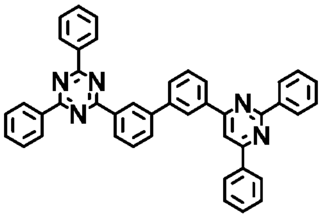
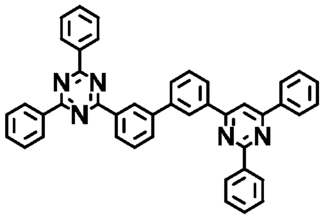

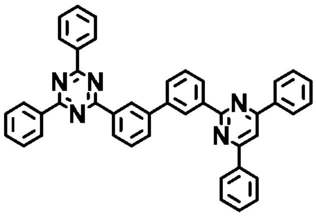
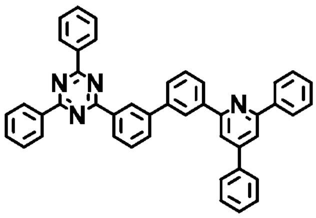
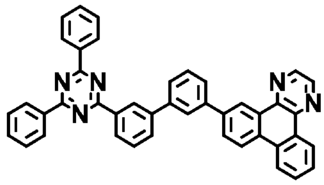

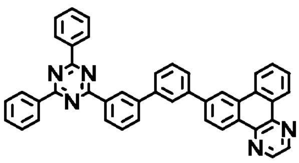
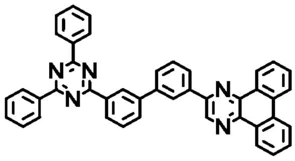
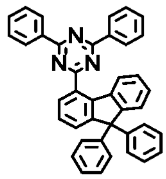
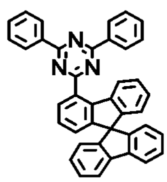

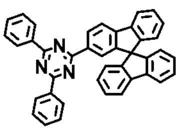
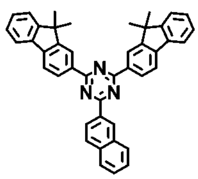
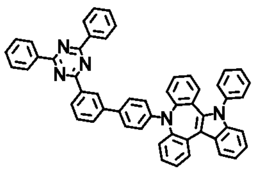

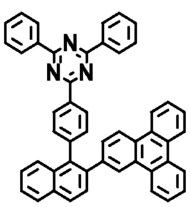
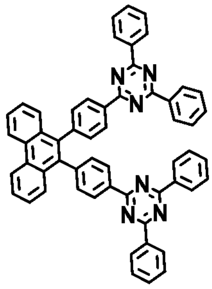
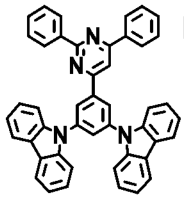
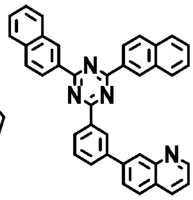

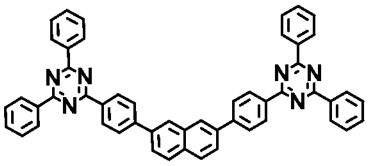
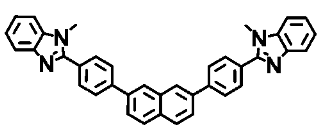

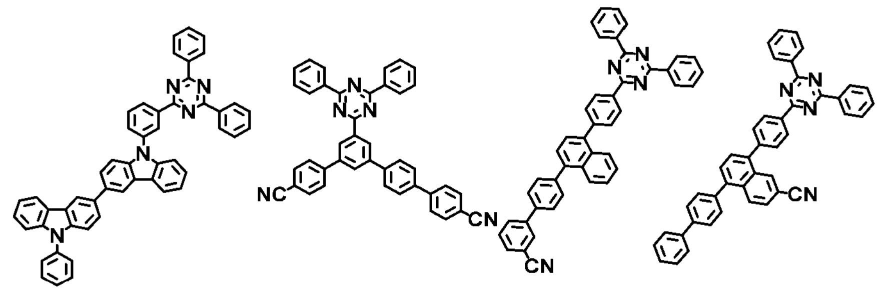

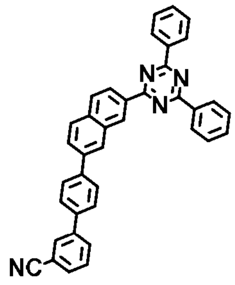

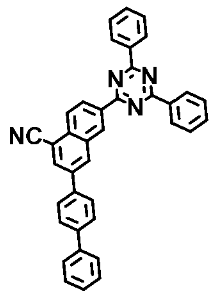

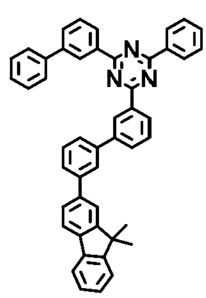

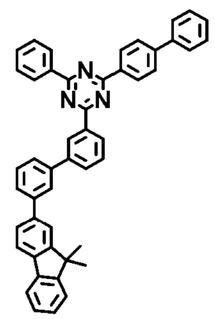

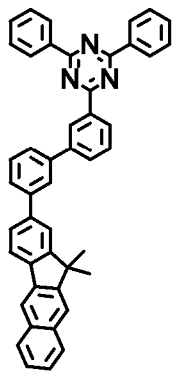

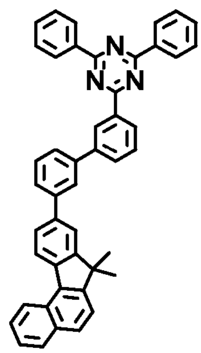

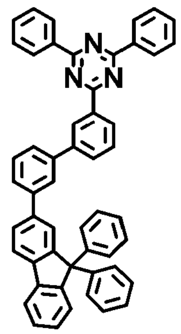

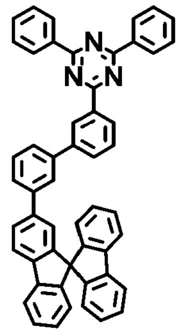

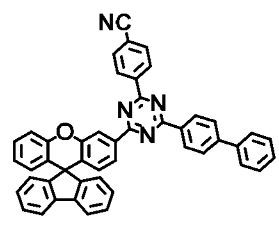

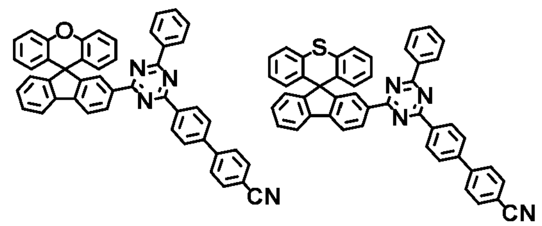

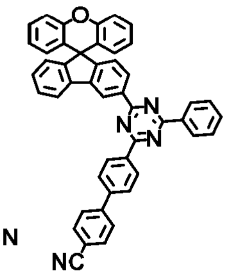

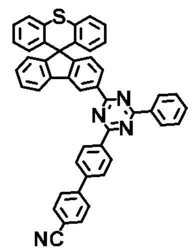

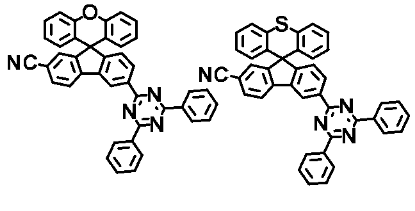

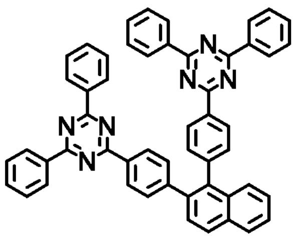
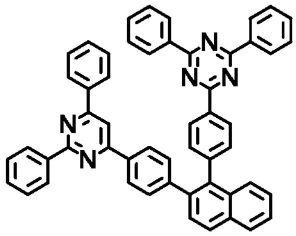
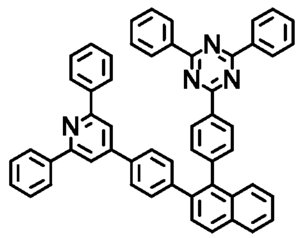

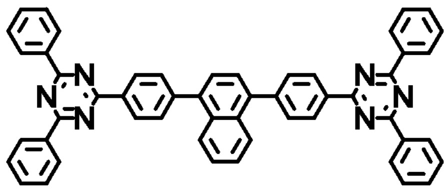
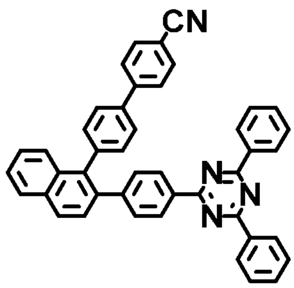

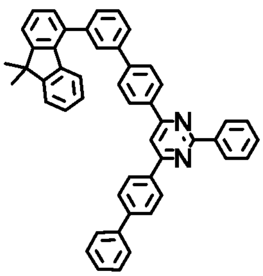
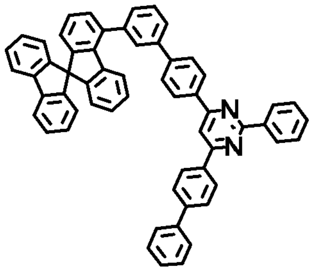
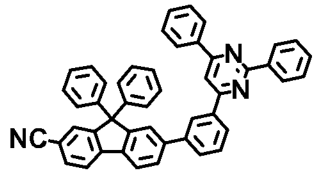

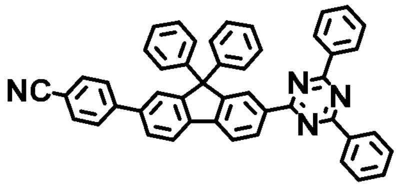
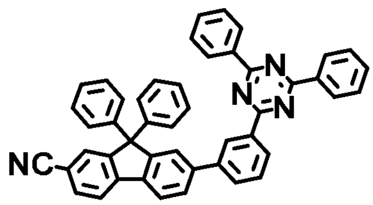

An embodiment may provide an organic light emitting diode including the light emitting layer as the organic layer.

Another embodiment may provide an organic light emitting diode including a light emitting layer and a hole transport region as the organic layer.

Another embodiment may provide an organic light emitting diode including a light emitting layer and an electron transport region as the organic layer.

Another embodiment of the present invention may provide an organic light emitting diode including a hole transport region 140 and an electron transport region 150 in addition to the light emitting layer 130 as the organic layer 105, as shown in FIG. 1.

On the other hand, an organic light emitting diode may further include an electron injection layer (not shown), a hole injection layer (not shown), etc. in addition to the light emitting layer as the organic layer.

The organic light emitting diodes 100 may be manufactured by forming an anode or a cathode on a substrate, and then forming an organic layer by a dry film method such as vacuum deposition, sputtering, plasma plating and ion plating, and forming a cathode or an anode thereon.

The aforementioned organic light emitting diode may be applied to an organic light emitting display device.

## DETAILED DESCRIPTION OF THE EMBODIMENTS

Hereinafter, the embodiments are illustrated in more detail with reference to examples. However, these examples are exemplary, and the scope of claims is not limited thereto.

Hereinafter, starting materials and reactants used in Examples and Synthesis Examples were purchased from Sigma-Aldrich Co. Ltd., TCI Inc., Tokyo chemical industry, or P&H tech as far as there in no particular comment or were

synthesized by known methods.

**(Preparation of Compound for Organic Optoelectronic Device)**

The compounds presented as a more specific example of the compound of the present invention were synthesized through the following steps.

**Synthesis Example 1: Synthesis of Compound A-1**

[Reaction Scheme 1]

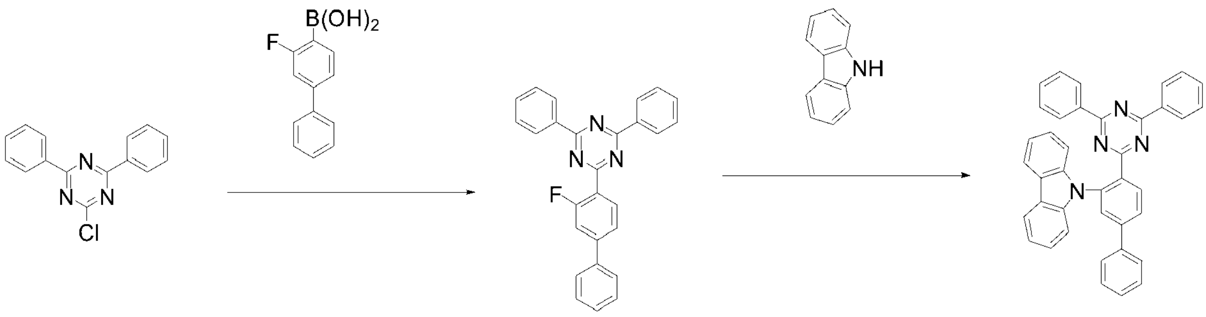

P-1

A-1

1st step: Synthesis of Intermediate P-1

2-chloro-4,6-diphenyl-1,3,5-triazine (50 g / 1.0 eq.), (3-fluoro-[1,1'-biphenyl]-4-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 60 g of Intermediate P-1 was obtained through column chromatography.

2nd step: Synthesis of Compound A-1

Intermediate P-1 (30 g / 1.0 eq.), 9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 32 g of Compound A-1 was obtained through column chromatography.

**Synthesis Example 2: Synthesis of Compound A-3**

[Reaction Scheme 2]

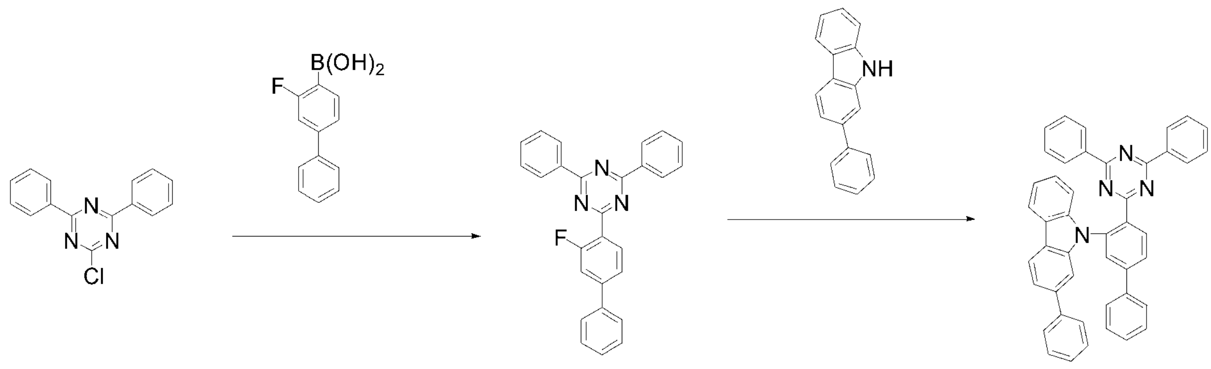

P-1 A-3

1st step: Synthesis of Intermediate P-1

60 g of Intermediate P-1 was obtained in the same manner as in the 1st step of Synthesis Example 1.

2nd step: Synthesis of Compound A-3

Intermediate P-1 (30 g / 1.0 eq.), 2-phenyl-9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 38 g of Compound A-3 was obtained through column chromatography.

**Synthesis Example 3: Synthesis of Compound A-11**

[Reaction Scheme 3]

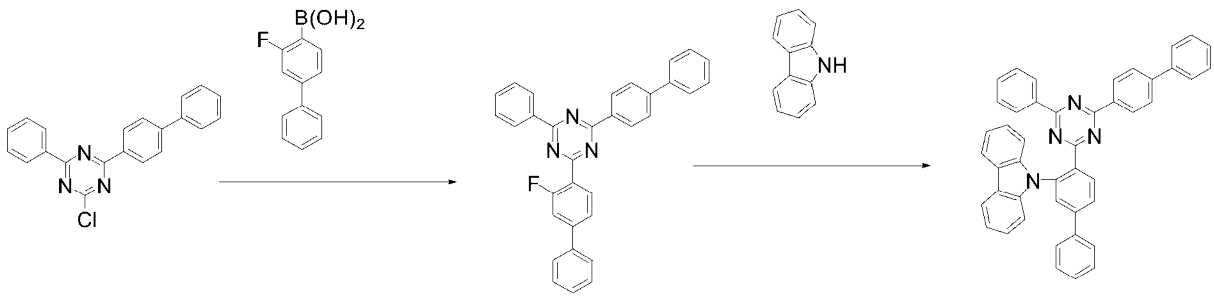

P-2 A-11

1st step: Synthesis of Intermediate P-2

2-([1,1'-biphenyl]-4-yl)-4-chloro-6-phenyl-1,3,5-triazine (30 g / 1.0 eq.), (3-fluoro-[1,1'-biphenyl]-4-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 33 g of Intermediate P-2 was obtained through column chromatography.

2nd step: Synthesis of Compound A-11

Intermediate P-2 (33 g / 1.0 eq.), 9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 25 g of Compound A-11 was obtained through column

chromatography.

**Synthesis Example 4: Synthesis of Compound A-21**

[Reaction Scheme 4]

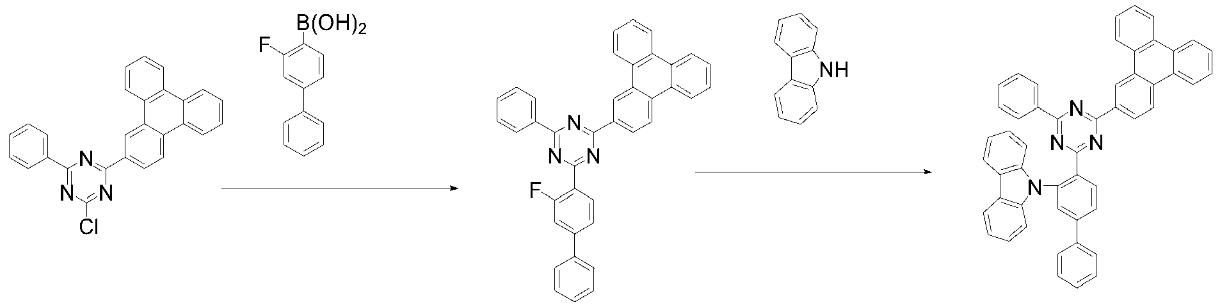

P-3

A-21

1st step: Synthesis of Intermediate P-3

2-chloro-4-phenyl-6-(triphenylen-2-yl)-1,3,5-triazine (40 g / 1.0 eq.), (3-fluoro-[1,1'-biphenyl]-4-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 42 g of Intermediate P-3 was obtained through column chromatography.

2nd step: Synthesis of Compound A-21

Intermediate P-3 (42 g / 1.0 eq.), 9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 32 g of Compound A-21 was obtained through column chromatography.

**Synthesis Example 5: Synthesis of Compound B-136**

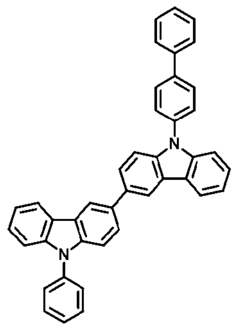

B-136

Compound B-136 was synthesized by referring to the synthesis method known in the US 10476008 B2 registered patent.

**Comparative Synthesis Example 1: Synthesis of Compound R-1**

[Reaction Scheme 5]

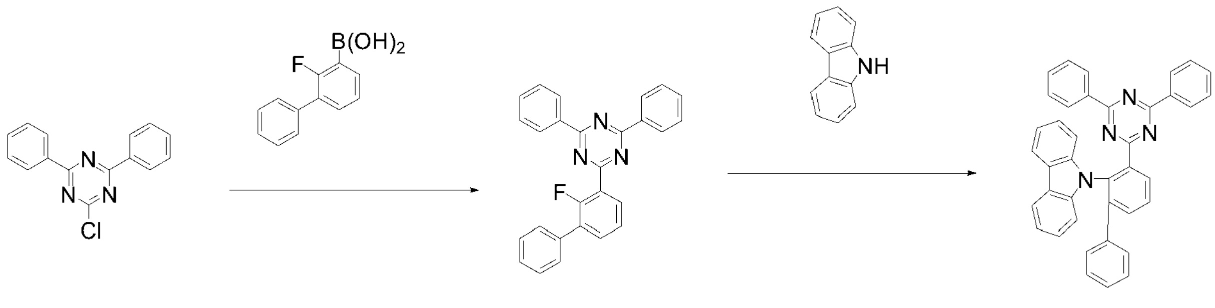

P-4 R-1

1st step: Synthesis of Intermediate P-4

2-chloro-4,6-diphenyl-1,3,5-triazine (30 g / 1.0 eq.), (2-fluoro-[1,1'-biphenyl]-3-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 26 g of Intermediate P-4 was obtained through column chromatography.

2nd step: Synthesis of Compound R-1

Intermediate P-4 (26 g / 1.0 eq.), 9H-carbazole (1.5 eq.), $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 20 g of Compound R-1 was obtained through column chromatography.

**Comparative Synthesis Example 2: Synthesis of Compound R-2**

[Reaction Scheme 6]

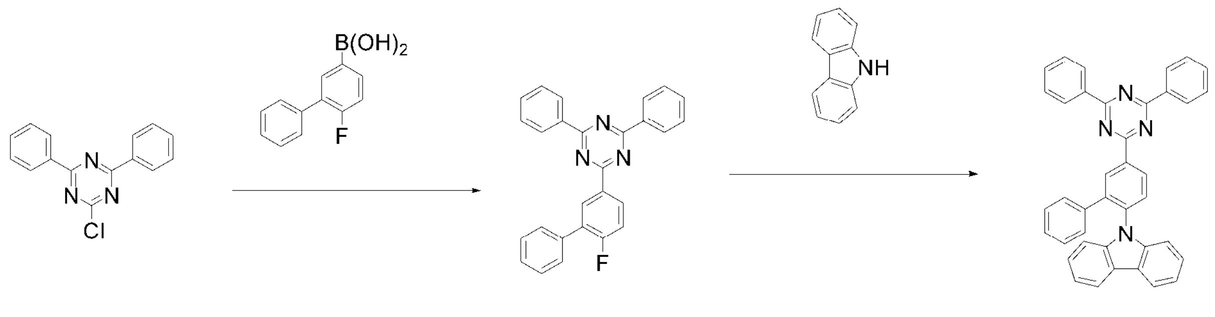

P-5 R-2

1st step: Synthesis of Intermediate P-5

2-chloro-4,6-diphenyl-1,3,5-triazine (30 g / 1.0 eq.), (6-fluoro-[1,1'-biphenyl]-3-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 26 g of Intermediate P-5 was obtained through column chromatography.

2nd step: Synthesis of Compound R-2

Intermediate P-5 (26 g / 1.0 eq.), 9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 15 g of Compound R-2 was obtained through column chromatography.

**Comparative Synthesis Example 3: Synthesis of Compound R-3**

[Reaction Scheme 7]

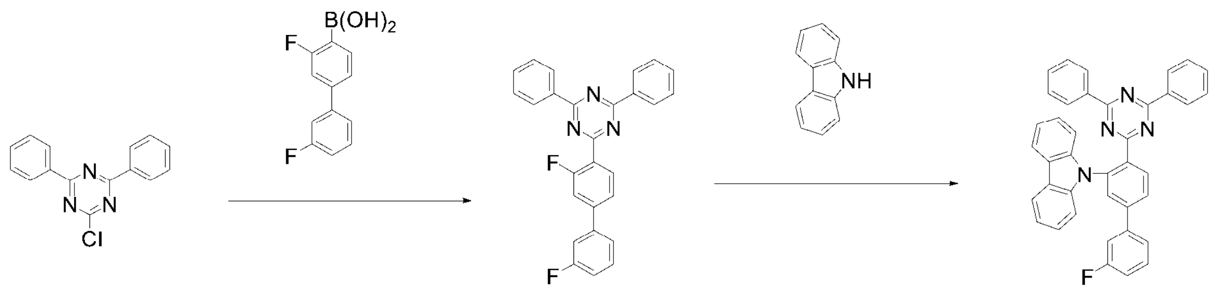

P-6 R-3

1st step: Synthesis of Intermediate P-6

2-chloro-4,6-diphenyl-1,3,5-triazine (30 g / 1.0 eq.), (3,3'-difluoro-[1,1'-biphenyl]-4-yl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 30 g of Intermediate P-6 was obtained through column chromatography.

2nd step: Synthesis of Compound R-3

Intermediate P-6 (30 g / 1.0 eq.), 9H-carbazole (1.2 eq.), and $K_3PO_4$ (2.5 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 8 g of Compound R-3 was obtained through column chromatography.

**Comparative Synthesis Example 4: Synthesis of Compound R-4**

[Reaction Scheme 8]

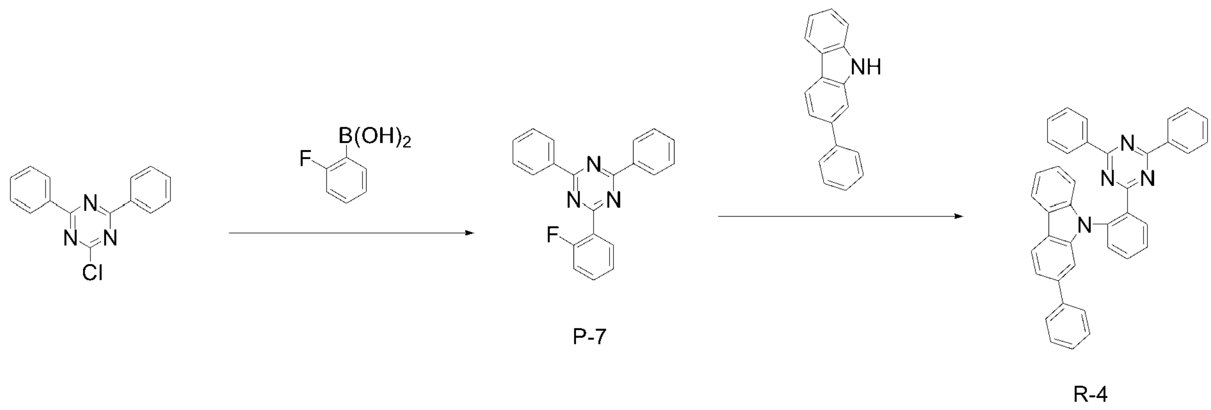


1st step: Synthesis of Intermediate P-7

2-chloro-4,6-diphenyl-1,3,5-triazine (30 g / 1.0 eq.), (2-fluorophenyl)boronic acid (1.1 eq.), $Pd(PPh_3)_4$ (0.05 eq.), and $K_2CO_3$ (3.0 eq.) with THF (500 mL) and distilled water (165 mL) were injected into a flask and refluxed at 80 °C. After 12 hours, the reaction was completed, the resultant was diluted with DCM, washed three times with brine, and dried with $MgSO_4$. Subsequently, 25 g of Intermediate P-7 was obtained through column chromatography.

2nd step: Synthesis of Compound R-4

Intermediate P-7 (25 g / 1.0 eq.), 2-phenyl-9H-carbazole (1.5 eq.), and $K_3PO_4$ (3.0 eq.) with DMF (500 mL) were injected into a flask and refluxed at 150 °C. After 12 hours, the reaction was completed, the resultant was diluted with DCM, washed three times with brine, and dried with $MgSO_4$. Subsequently, 20 g of Compound R-4 was obtained through column chromatography.

**(Manufacturing of Organic Light Emitting Diode)**

**Example 1**

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A is deposited on the hole injection layer to a thickness of 1350 Å to form a hole transport layer. Compound B was deposited on the hole transport layer to a thickness of 350 Å to form a hole transport auxiliary layer. Compound A-1 obtained in Synthesis Example 1 was used as a host, and 7 wt% of PhGD was doped as a dopant to form a 400Å-thick light emitting layer on the hole transport auxiliary layer by vacuum deposition. Subsequently, Compound C was deposited on the light emitting layer to a thickness of 50 Å to form an electron transport auxiliary layer, and Compound D and Liq were simultaneously vacuum-deposited in a weight ratio of 1:1 to form a 300 Å-thick electron transport layer. An organic light emitting diode was manufactured by sequentially vacuum-depositing 15 Å of LiQ and 1,200 Å of Al on the electron transport layer to form a cathode.

ITO / Compound A (1% NDP-9 doping, 100 Å) / Compound A (1350 Å) / Compound B (350 Å) / EML [93 wt% of host (Compound A-1) : 7 wt% of PhGD] (400 Å) / Compound C (50 Å) / Compound D : LiQ (300 Å) / LiQ (15 Å) / Al (1200 Å).

Compound A: N-(biphenyl-4-yl)-9,9-dimethyl-N-(4-(9-phenyl-9H-carbazol-3-yl)phenyl)-9H-fluoren-2-amine
Compound B: N-[4-(4-dibenzofuranyl)phenyl]-N-[4-(9-phenyl-9H-fluoren-9-yl)phenyl][1,1'-biphenyl]-4-amine
Compound C: 2,4-diphenyl-6-(4',5',6'-triphenyl[1,1':2',1":3",1‴:3‴,1⁗-quinquephenyl]-3⁗-yl)-1,3,5-triazine
Compound D: 2-(1,1'-biphenyl-4-yl)-4-(9,9-diphenylfluoren-4-yl)-6-phenyl-1,3,5-triazine

[PhGD]

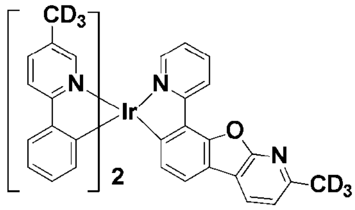

**Examples 2 to 4 and Comparative Examples 1 to 4**

Each diode of Examples 2 to 4 and Comparative Examples 1 to 4 was manufactured in the same manner as Example 1, except that the host was changed as shown in Table 1.

**Example 5**

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A is deposited on the hole injection layer to a thickness of 1350 Å to form a hole transport layer. Compound E was deposited on the hole transport layer to a thickness of 350 Å to form a hole transport auxiliary layer. Compound A-1 obtained in Synthesis Example 1 and Compound B-136 obtained in Synthesis Example 4 were simultaneously used as a host and 10 wt% of PhGD was used as a dopant to form a 400 Å-thick light emitting layer on the hole transport auxiliary layer by vacuum deposition. Compound A-1 and Compound B-136 were used in a weight ratio of 3:7. Subsequently, Compound F was deposited on the light emitting layer to a thickness of 50 Å to form an electron transport auxiliary layer, and Compound G and Liq were simultaneously vacuum-deposited in a weight ratio of 1:1 to form a 300 Å-thick electron transport layer. An organic light emitting diode was manufactured by sequentially vacuum-depositing 15 Å of LiQ and 1,200 Å of Al on the electron transport layer to form a cathode.

ITO / Compound A (3% NDP-9 doping, 100 Å) / Compound A (1350 Å) / Compound E (350 Å) / EML [host (Compound A-1: Compound B-136 = 3:7) : PhGD = 90 wt%:10 wt%] (400 Å) / Compound F (50 Å) / Compound G: LiQ 300Å / LiQ (300 Å) / LiQ (15 Å) / Al (1200 Å).

Compound A: N-(biphenyl-4-yl)-9,9-dimethyl-N-(4-(9-phenyl-9H-carbazol-3-yl)phenyl)-9H-fluoren-2-amine
Compound E: N,N-bis(9,9-dimethyl-9H-fluoren-4-yl)-9,9-spirobi(fluorene)-2-amine
Compound F: 2-[3'-(9,9-dimethyl-9H-fluoren-2-yl)[1,1'-biphenyl]-3-yl]-4,6-diphenyl-1,3,5-triazine
Compound G: 2-[4-[4-(4'-cyano-1,1'-biphenyl-4-yl)-1-naphthyl]phenyl]-4,6-diphenyl-1,3,5-triazine

**Examples 6 to 8 and Comparative Examples 5 to 8**

Each diode of Examples 6 to 8 and Comparative Examples 5 to 8 was manufactured in the same manner as Example 5, except that the host was changed as shown in Table 2.

**Evaluation**

(1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

(2) Measurement of Luminance Change Depending on Voltage Change

Luminance was measured by using a luminance meter (Minolta Cs-1000A), while the voltage of the organic light emitting diodes was increased from 0 V to 10 V.

(3) Measurement of Luminous Efficiency

Luminous efficiency (cd/A) at the same current density (10 mA/$cm^2$) were calculated by using the luminance and current density from (1) and (2) above.
Relative values based on the luminous efficiency of Comparative Example 1 are shown in Table 1.
Relative values based on the luminous efficiency of Comparative Example 5 are shown in Table 2.

(4) Measurement of Life-span

T95 life-spans of the organic light emitting diodes were measured as a time when their luminance decreased down to 95% relative to the initial luminance (cd/$m^2$) after emitting light with the initial luminance of 2,4000 cd/$m^2$ and measuring their luminance decrease depending on time with a Polanonix life-span measurement system.
Relative values based on the T95 life-span of Comparative Example 1 are shown in Table 1.
Relative values based on the T95 life-span of Comparative Example 5 are shown in Table 2.

(5) Measurement of Driving Voltage

The driving voltage of each diode were measured at 15 mA/$cm^2$ using a current-voltmeter (Keithley 2400), and the results were obtained.
Relative values based on the driving voltage of Comparative Example 1 are shown in Table 1.

(Table 1)

| | Host | Driving voltage (%) | Luminous efficiency (%) | Life-spanT95 (%) |
|---|---|---|---|---|
| Example 1 | A-1 | 98.2 | 101.9 | 145.6 |
| Example 2 | A-3 | 97.9 | 102.5 | 162.3 |
| Example 3 | A-11 | 96.5 | 103.2 | 181.2 |
| Example 4 | A-21 | 95.3 | 103.0 | 210.2 |
| Comparative Example 1 | R-1 | 100.0 | 100.0 | 100.0 |
| Comparative Example 2 | R-2 | 103.2 | 98.5 | 95.2 |
| Comparative Example 3 | R-3 | 101.1 | 100.2 | 11.4 |
| Comparative Example 4 | R-4 | 101.2 | 100.0 | 105.6 |

(Table 2)

| | First host | Second host | Luminous efficiency (%) | Life-span T95 (%) |
|---|---|---|---|---|
| Example 5 | A-1 | B-136 | 102.6 | 161.9 |
| Example 6 | A-3 | B-136 | 104.4 | 184.5 |
| Example 7 | A-11 | B-136 | 104.8 | 195.2 |
| Example 8 | A-21 | B-136 | 104.3 | 220.5 |
| Comparative Example 5 | R-1 | B-136 | 100.0 | 100.0 |
| Comparative Example 6 | R-2 | B-136 | 96.5 | 92.2 |
| Comparative Example 7 | R-3 | B-136 | 100.5 | 5.6 |
| Comparative Example 8 | R-4 | B-136 | 100.3 | 107.3 |

Referring to Table 1, the organic light emitting diodes to which the compounds according to Examples of the present invention are applied have significantly improved driving, efficiency, and life-span characteristics compared to the organic light emitting diodes according to Comparative Examples, and in particular, referring to Table 2, the efficiency and life-span characteristics of the organic light emitting diodes to which the composition including the compound according to Examples of the present invention is applied are also improved.
While this invention has been described in connection with what is presently considered to be practical example

embodiments, it is to be understood that the invention is not limited to the disclosed embodiments. On the contrary, it is intended to cover various modifications and equivalent arrangements included within the spirit and scope of the appended claims.

## Claims

**1.** A compound for an organic optoelectronic device represented by Chemical Formula 1:

[Chemical Formula 1]

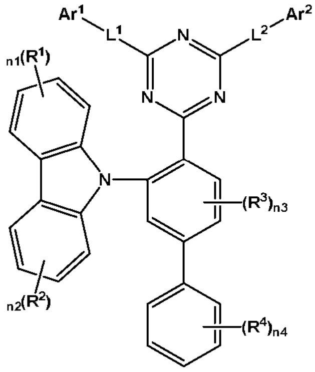

wherein, in Chemical Formula 1,

$Ar^1$ and $Ar^2$ are each independently a substituted or unsubstituted C2 to C30 heterocyclic group,
$L^1$ and $L^2$ are each independently a single bond, a substituted or unsubstituted C6 to C30 arylene group, or a substituted or unsubstituted C2 to C30 heteroarylene group,
$R^1$ and $R^2$ are each independently hydrogen, deuterium, or a substituted or unsubstituted C6 to C30 aryl group,
$R^3$ and $R^4$ are each independently hydrogen or deuterium,
n1, n2 and n4 are each independently one of integers of 1 to 4, and
n3 is one of integers of 1 to 3.

**2.** The compound for an organic optoelectronic device as claimed in claim 1, wherein

the compound is represented by any one of Chemical Formula 1-1 to Chemical Formula 1-4:

[Chemical Formula 1-1] [Chemical Formula 1-2]

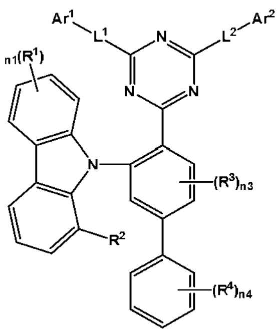

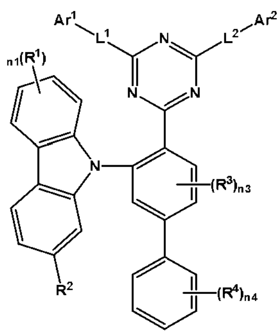

[Chemical Formula 1-3] [Chemical Formula 1-4]

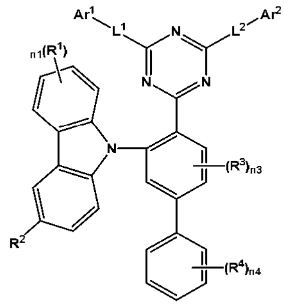
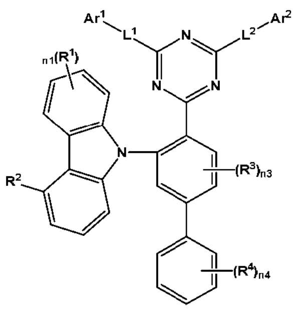

wherein, in Chemical Formula 1-1 to Chemical Formula 1-4,
$Ar^1$ to $Ar^3$, $R^1$, $R^2$, n1, n3, n4, $L^1$, and $L^2$ are the same as defined in claim 1.

**3.** The compound for an organic optoelectronic device as claimed in claim 1, wherein
$R^1$ and $R^2$ are each independently hydrogen, deuterium, a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, or a substituted or unsubstituted triphenylene group.

**4.** The compound for an organic optoelectronic device as claimed in claim 1, wherein
$Ar^1$ and $Ar^2$ are each independently a substituted or unsubstituted carbazolyl group, a substituted or unsubstituted dibenzofuranyl group, or a substituted or unsubstituted dibenzothiophenyl group.

**5.** The compound for an organic optoelectronic device as claimed in claim 1, wherein

*-$L^1$-$Ar^1$ and *-$L^2$-$Ar^2$ are each independently selected from the substituents listed in Group I :

[Group Ⅰ]

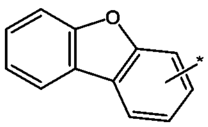
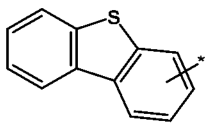
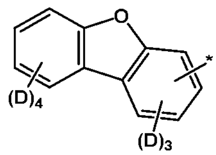
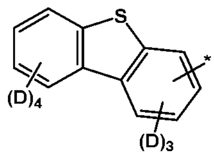
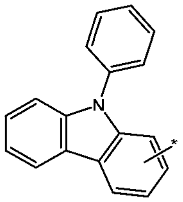
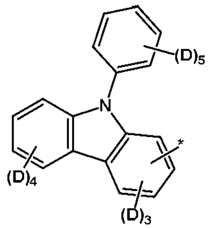

wherein, in Group I, * is a linking point.

**6.** The compound for an organic optoelectronic device as claimed in claim 1, wherein
the compound is one selected from the compounds listed in Group 1:

[Group 1]

[A-26] 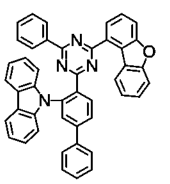 [A-27] 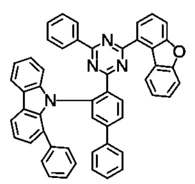 [A-28] 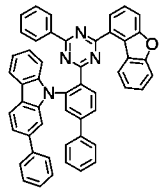 [A-29] 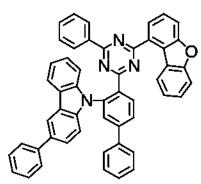 [A-30] 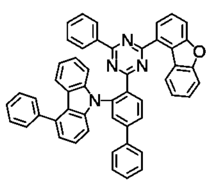

[A-31] 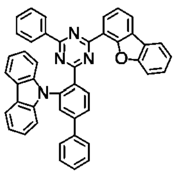 [A-32] 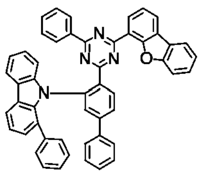 [A-33] 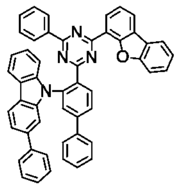 [A-34] 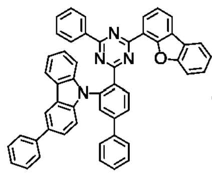 [A-35] 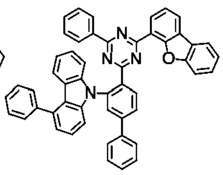

[A-36] 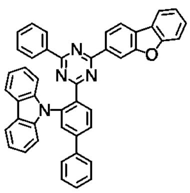 [A-37] 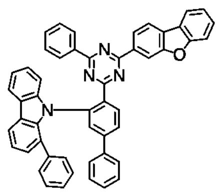 [A-38] 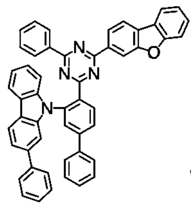 [A-39] 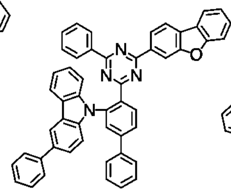 [A-40] 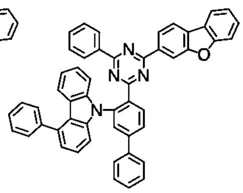

[A-41] 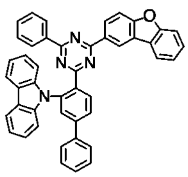 [A-42] 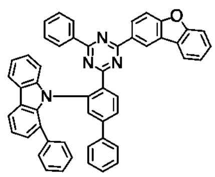 [A-43] 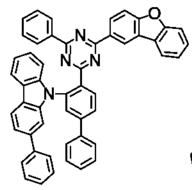 [A-44] 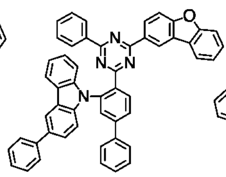 [A-45] 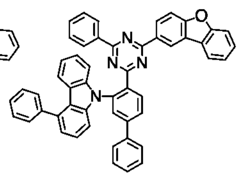

[A-46] 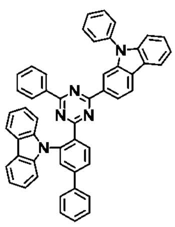 [A-47] 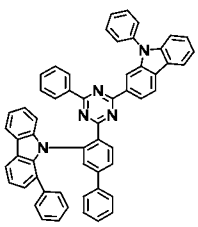 [A-48] 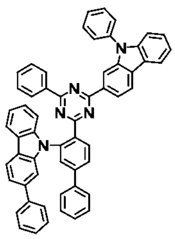 [A-49] 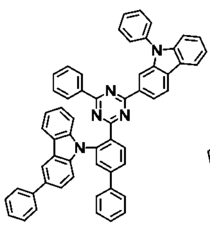 [A-50] 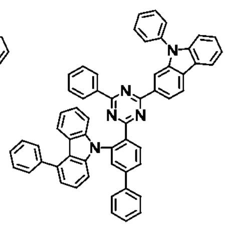.

**7.** A composition for an organic optoelectronic device, comprising

a first compound and a second compound,
wherein the first compound is the compound for an organic optoelectronic device as claimed in claim 1, and the second compound is a compound for an organic optoelectronic device represented by Chemical Formula 2; a compound for an organic optoelectronic device represented by a combination of Chemical Formula 3 and Chemical Formula 4:

[Chemical Formula 2]

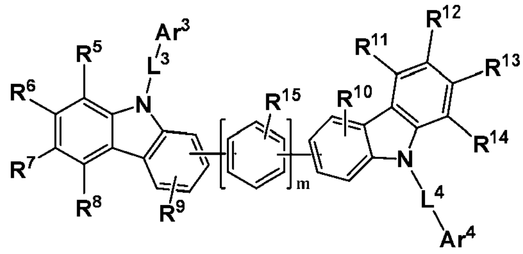

wherein, in Chemical Formula 2,
$Ar^3$ and $Ar^4$ are each independently a substituted or unsubstituted C6 to C20 aryl group or a substituted or unsubstituted C2 to C30 heterocyclic group,
$L^3$ and $L^4$ are each independently a single bond or a substituted or unsubstituted C6 to C20 arylene group,
$R^5$ to $R^{15}$ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group, and
m is one of integers of 0 to 2;

[Chemical Formula 3] [Chemical Formula 4]

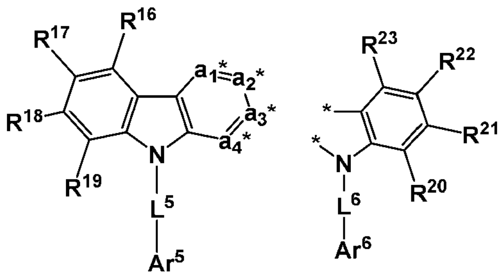

wherein, in Chemical Formulas 3 and 4,
$Ar^5$ and $Ar^6$ are each independently a substituted or unsubstituted C6 to C20 aryl group or a substituted or unsubstituted C2 to C30 heterocyclic group,
among $a_1$* to $a_4$* in Chemical Formula 3, two adjacent ones are each linking carbon (C) linked to * in Chemical Formula 4,
among $a_1$* to $a_4$* in Chemical Formula 3, the remaining two not linked to * in Chemical Formula 4 are each independently C-$L^a$-$R^a$,
$L^a$, $L^5$, and $L^6$ are each independently a single bond or a substituted or unsubstituted C6 to C20 arylene group, and
$R^2$ and $R^{16}$ to $R^{23}$ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group.

**8.** The composition for an organic optoelectronic device as claimed in claim 7, wherein

Chemical Formula 2 is represented by Chemical Formula 2-8:

[Chemical Formula 2-8]

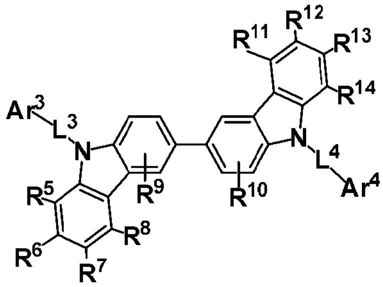

wherein, in Chemical Formula 2-8,
$R^5$ to $R^{14}$ are each independently hydrogen, deuterium, or a substituted or unsubstituted C6 to C12 aryl group, and
*-$L^3$-$Ar^3$ and *-$L^4$-$Ar^4$ are each independently selected from the substituents listed in Group II,

[Group Ⅱ]

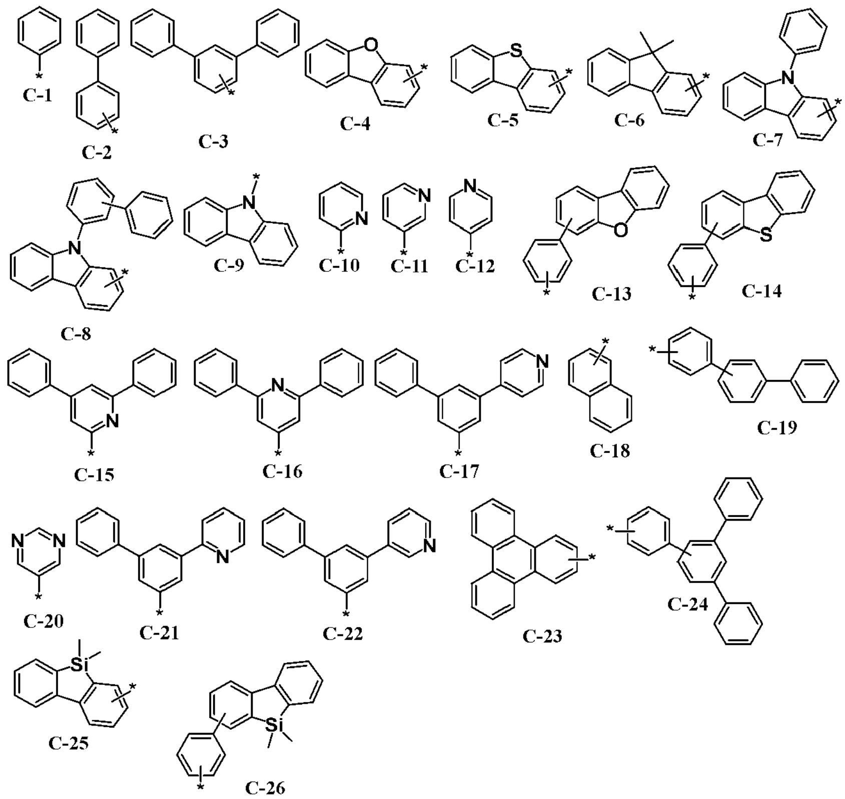

C-1 C-2 C-3 C-4 C-5 C-6 C-7

C-8 C-9 C-10 C-11 C-12 C-13 C-14

C-15 C-16 C-17 C-18 C-19

C-20 C-21 C-22 C-23 C-24

C-25 C-26

wherein, in Group II, * is a linking point.

**9.** The composition for an organic optoelectronic device as claimed in claim 7, wherein

the combination of Chemical Formula 3 and Chemical Formula 4 is represented by Chemical Formula 3C:

[Chemical Formula 3C]

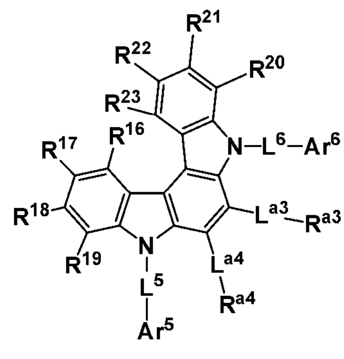

wherein, in Chemical Formula 3C,
$L^{a3}$ and $L^{a4}$ are a single bond,
$L^5$ and $L^6$ are each independently a single bond or a substituted or unsubstituted C6 to C12 arylene group,
$R^{16}$ to $R^{23}$, $R^{a3}$ and $R^{a4}$ are each independently hydrogen or a C6 to C12 aryl group, and
$Ar^5$ and $Ar^6$ are each independently a substituted or unsubstituted phenyl group, a substituted or unsubstituted

naphthyl group or a substituted or unsubstituted biphenyl group.

**10.** An organic photoelectronic device, comprising

an anode and a cathode facing each other, and
at least one organic layer between the anode and the cathode,
wherein the organic layer includes the compound for an organic optoelectronic device as claimed in any one of claim 1 to claim 6; or the composition for an organic optoelectronic device as claimed in any one of claim 7 to claim 9.

**11.** The organic photoelectronic device as claimed in claim 10, wherein

the organic layer includes a light emitting layer, and
the light emitting layer includes the compound for an organic optoelectronic device or composition for an organic optoelectronic device.

**12.** A display device comprising the organic photoelectronic device as claimed in claim 10.

【Figure 1】

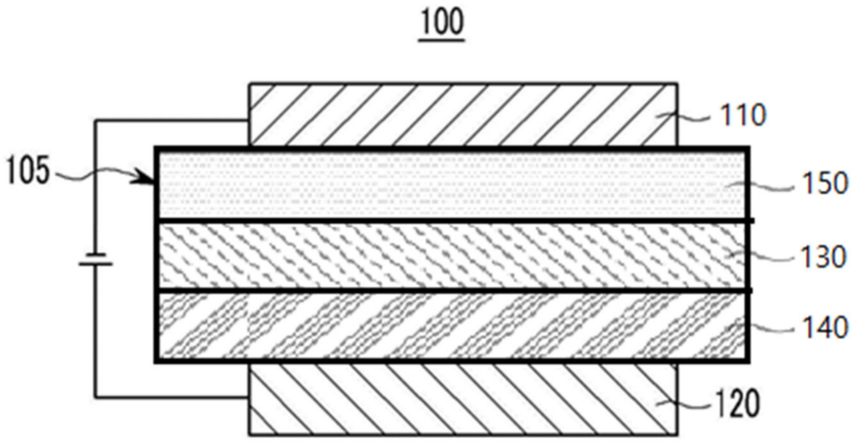

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5061569 A
- JP 5009471 A
- WO 1995009147 A1
- JP 7126615 A
- JP 10095973 A
- US 10476008 B2